(19) Europäisches Patentamt
European Patent Office
Office européen des brevets

(11) EP 2 794 711 B1

(12) # EUROPÄISCHE PATENTSCHRIFT

(45) Veröffentlichungstag und Bekanntmachung des Hinweises auf die Patenterteilung:
**10.06.2020 Patentblatt 2020/24**

(21) Anmeldenummer: **12801748.0**

(22) Anmeldetag: **17.12.2012**

(51) Int Cl.:
***C08G 18/48*** (2006.01) ***C08G 18/10*** (2006.01)
***C08G 18/38*** (2006.01) ***C08G 63/91*** (2006.01)
***C08G 18/42*** (2006.01) ***C08G 63/676*** (2006.01)

(86) Internationale Anmeldenummer:
**PCT/EP2012/075819**

(87) Internationale Veröffentlichungsnummer:
**WO 2013/092501 (27.06.2013 Gazette 2013/26)**

(54) **HYDROXY-AMINOPOLYMERE UND VERFAHREN ZU DEREN HERSTELLUNG**

HYDROXY AMINOPOLYMERS AND METHOD FOR THERE MANUFACTURE

HYDROXY-AMINOPOLYMÈRES ET LEUR PROCÉDÉ DE FABRICATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.12.2011 EP 11194419**

(43) Veröffentlichungstag der Anmeldung:
**29.10.2014 Patentblatt 2014/44**

(73) Patentinhaber: **Covestro Deutschland AG**
**51373 Leverkusen (DE)**

(72) Erfinder:
- **LORENZ, Klaus**
  **41539 Dormagen (DE)**
- **HOFMANN, Jörg**
  **47800 Krefeld (DE)**
- **NEFZGER, Hartmut**
  **50259 Pulheim (DE)**

(74) Vertreter: **Levpat**
**c/o Covestro AG**
**Gebäude 4825**
**51365 Leverkusen (DE)**

(56) Entgegenhaltungen:
**EP-A1- 1 493 767 US-A- 5 554 687**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Hydroxy-Aminopolymere, Verfahren zu deren Herstellung sowie die Verwendung solcher Hydroxy-Aminopolymere zur Herstellung von Polyurethanharnstoffen.

Polymere, welche sowohl aminfunktionell sind als auch Hydroxylgruppen tragen (sogenannte Hydroxy-Aminopolymere), stoßen in bestimmten Anwendungsgebieten, speziell im Bereich der Polyurethanindustrie, auf zunehmendes Interesse. Der Grund hierfür liegt darin, dass sich durch die Gegenwart zweier unterschiedlicher Typen funktioneller Gruppen, nämlich der Aminfunktionalitäten sowie der Hydroxylgruppen, neuartige Eigenschafts- und Verarbeitungsprofile erzielen lassen. So besteht beispielsweise durch die Kombination der gegenüber Isocyanatgruppen deutlich reaktiveren Aminogruppen mit den weniger reaktiven Hydroxylgruppen die Möglichkeit, den zeitlichen Verlauf von Aushärteprozessen in gewünschter Weise zu beeinflussen, was bislang bei Gegenwart nur eines Typs der vorgenannten isocyanatreaktiven funktionellen Gruppen nicht oder nur in beschränktem Maße möglich ist.

Im Allgemeinen kann die Aminofunktionalität von Hydroxy-Aminopolymeren über die Addition von primären Aminen oder Ammoniak an elektronenarme Doppelbindungen, beispielsweise von (Meth-)Acylattyp, in Makromoleküle eingeführt werden. Die Addition von Aminen an (meth-)acrylatgruppenhaltige Polymere, u. a. an (meth-)acrylat-gruppenhaltige Polyether, ist an sich bekannt, beispielsweise werden solche Verfahren in US 5,739,192 A1, US 5,597,390 A1, US 2005/0171002 A1, DE 196 16 984 A1, DE 195 08 308 A1, WO 2010/090345 A1, JP 2009/22753 A1 und JP 04089860 A1 erwähnt.

Demgegenüber ist der Erhalt der die elektronenarmen Doppelbindungen enthaltenden Vorläuferverbindungen im Stand der Technik entweder nicht beschrieben oder erfolgt über nach statistischen Gesetzmäßigkeiten ablaufenden Kondensationsreaktionen, beispielsweise über die Veresterung von Acrylsäure mit difunktionellen Polyethern oder die Umsetzung von Acryloylchlorid mit difunktionellen Polyethern.

Allen beschriebenen Verfahren ist gemein, dass die Einführung der Doppelbindungen in die Vorläuferverbindungen der Hydroxy-Aminopolymere auf Kosten der Zahl der Hydroxyfunktionen erfolgt. Somit erlauben diese Verfahren es nicht, die ursprüngliche Hydroxyfunktionalität, die bei Polyethermolekülen im allgemeinen durch die Funktionalität der zur Herstellung der Polyether verwendeten Startermoleküle gegeben ist, während der Einführung der Aminofunktionen zu erhalten.

Verfahren, wie sie beispielsweise in US 4,874,837 A1 beschrieben sind, lösen dieses Problem zum Teil dadurch, dass Mischungen aus Maleinsäureanhydrid und weiteren Anhydriden mit den Hydroxygruppen von niedermolekularen Polyetherpolyolen zur Reaktion gebracht werden, die entstehenden Säuregruppen der Halbester durch Addition von Alkylenoxiden in Hydroxygruppen rückverwandelt werden und die Aminfunktion durch Addition von primären oder sekundären Aminogruppen enthaltenden Aminoalkoholen oder primären bzw. sekundären Aminogruppen enthaltenden Diaminen an die reaktiven Doppelbindungen des Hydroxymaleats eingeführt wird.

Ein struktureller Nachteil der auf diese Weise hergestellten Hydroxy-Aminopolyetherester liegt darin, dass die Hydroxygruppen und die Aminogruppen einen fixierten und geringen Abstand von 6 bis 7 kovalenten Bindungslängen voneinander aufweisen und pro Hydroxygruppe nur eine Aminogruppe eingeführt werden kann. Schematisch lässt sich dieser Sachverhalt wiedergeben wie folgt:

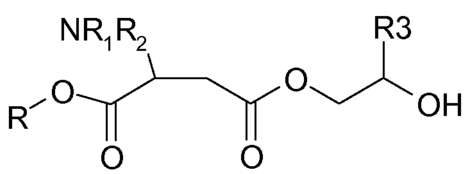
oder
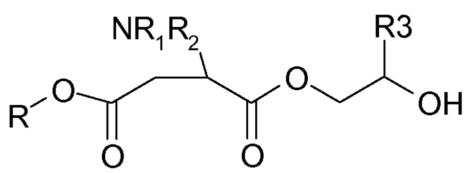

mit R = Polyetherrest, R1, R2 = Reste (Wasserstoff oder Alkyl) am Stickstoffatom und R3 = Rest (Wasserstoff oder Alkyl am zur Umwandlung der Säuregruppen in Hydroxygruppen eingesetzten Alkylenoxid)

Die beiden vorgenannten Produkte der Michael-Addition des Amins an die Doppelbindung liegen mehr oder weniger zu gleichen Teilen vor, wodurch sich eine Mischung von Polymeren mit 6 und mit 7 Bindungslängen zwischen Hydroxygruppe und Aminogruppe ergibt.

US 5,554,687 offenbart ein Verfahren, in welchem zunächst $\alpha,\beta$-ungesättigte Dicarbonsäuren oder deren Anhydride mit mehrwertigen Alkoholen oder Alkylenoxiden verestert werden, um ein ungesättigtes "Polyolpolyester-Präpolymer" zu erhalten. Die Veresterung soll im Falle von Maleinsäureanhydrid als $\alpha,\beta$-ungesättigtes Dicarbonsäureanhydrid vorzugsweise in Gegenwart von Morpholin als Isomerisierungskatalysator durchgeführt werden. Weitere Katalysatoren für den Veresterungsprozess nennt die Schrift nicht. In Gegenwart von Morpholin (ein aminischer Katalysator) reagiert Maleinsäureanhydrid mit Alkylenoxiden derart, dass zwischen zwei Maleinsäureanhydridbausteinen genau ein Alkylenoxidbaustein eingebaut wird. Wenn Ethylenoxid als Alkylenoxid eingesetzt wird, erhält man also letztlich einen Polyester wie aus der Reaktion von Maleinsäure mit Ethylenglykol.

Das "Polyolpolyester-Präpolymer" (a) wird dann in einem zweiten Schritt mit einem Polyoxyalkylenamin (b) in einem Gewichtsverhältnis (a)/(b) von 0,8 bis 50 zu einem aminhaltigen Polyesterharz umgesetzt. Als Polyoxyalkylenamine (b) werden Polymere aus Polyetherblöcken mit Aminoendgruppen offenbart, z. B. $H_2N\text{-}CHXCH_2\text{-}[OCH_2CHX]_n\text{-}NH_2$, wobei X für Wasserstoff oder eine Alkylgruppe mit 1 bis 18 Kohlenstoffatomen steht und n eine natürliche Zahl von 2 bis 70 ist. Die endständigen Aminogruppen addieren sich an die Doppelbindungen des Polyolpolyester-Präpolymers. Es entstehen also letztlich vernetzte Polyesterharze, in denen die Polyesterketten über Polyaminobrücken miteinander verknüpft sind. Ein Verfahren zur Herstellung von Hydroxy-Aminopolymeren im Sinne von Aminogruppen enthaltenden Poly(ether)esterpolyolen wird in US 5,554,687 weder beschrieben noch nahegelegt. Außerdem unterliegt das Verfahren von US 5,554,687 in Bezug auf die Struktur der Kettenenden den gleichen Einschränkungen wie US 4,874,837 A1. Der Abstand zwischen endständiger Hydroxygruppe und der ersten Aminogruppe kann hier ebenfalls maximal 7 kovalente Bindungslängen betragen.

Die Aufgabe der vorliegenden Erfindung bestand somit darin, ein Verfahren für die Herstellung von Hydroxy-Aminopolymeren zur Verfügung zu stellen, welches einerseits die Schaffung von Hydroxy-Aminopolymeren mit einem Abstand zwischen der Amino- und der Hydroxyfunktionalität von mehr als sieben kovalenten Bindungslängen erlaubt, andererseits soll die Möglichkeit eröffnet sein, mehr als nur eine Amingruppe pro OH-Gruppe in das Polymer einzubauen, dieses Verfahren weiterhin einfach in der Anwendung sein und außerdem die Entstehung von Nebenprodukten, wie beispielsweise Umesterungsprodukten möglichst vermeiden, so dass eine Aufarbeitung der Verfahrensprodukte in der Regel nicht notwendig ist. Die Aufgabe wird gelöst durch ein Verfahren zur Herstellung eines Hydroxy-Aminopolymers umfassend die Schritte:

a) Umsetzen einer wenigstens ein Zerewitinoff-aktives H-Atom tragenden H-funktionellen Starterverbindung mit einem ungesättigten cyclischen Carbonsäureanhydrid und wenigstens einer Alkylenoxidverbindung zum Erhalt eines Hydroxylgruppen tragenden Präpolymers,

b) Addition eines primären Amins und/oder von Ammoniak an die Doppelbindungen des nach Schritt a) erhaltenen Hydroxylgruppen tragenden Präpolymers zum Erhalt des Hydroxy-Aminopolymers,

wobei die Umsetzung der H-funktionellen Starterverbindung mit dem ungesättigten cyclischen Carbonsäureanhydrid und/ oder die Addition der Alkylenoxidverbindung unter Einsatz eines Doppelmetallcyanid-Katalysators (DMC-Katalysators) durchgeführt wird.

Dabei ist unter dem unbestimmten Artikel *"ein", "einer"* usw. zu verstehen, dass wahlweise jeweils auch mehrere dieser Komponenten in dem erfindungsgemäßen Verfahren miteinander umgesetzt werden können.

Ein *"Hydroxy-Aminopolymer"* im Sinne der vorliegenden Erfindung ist ein Aminogruppen enthaltendes Poly(ether)esterpolyol, d. h. ein Aminogruppen enthaltendes Polymer, in welchem Polyetherpolyolsequenzen über Dicarbonsäureester-Funktionalitäten miteinander verknüpft sind. Überraschenderweise hat sich herausgestellt, dass durch Addition von Aminen an Hydroxylgruppen tragende Präpolymere, erhältlich durch Umsetzung wenigstens eines Zerewitinoff-aktiven H-Atoms einer H-funktionellen Starterverbindung mit einem ungesättigten zyklischen Carbonsäureanhydrid und wenigstens einer Alkylenoxidverbindung unter Doppelmetallcyanid- (DMC-) Katalyse die vorgenannten Hydroxy-Aminopolymere erhältlich sind. Dabei kann das Verfahren so gesteuert werden, dass neben den bislang bekannten Hydroxy-Aminopolymeren mit wenigstens einem Abstand zwischen der Aminofunktion und der Hydroxylgruppe von sechs oder sieben kovalenten Bindungslängen auch solche Strukturen herstellbar sind, welche einen Abstand von größer als 7 kovalente Bindungslängen aufweisen. Das Verfahren kann beispielsweise auch derart ausgestaltet sein, dass nach Abreaktion einer dosierten Alkylenoxidverbindung ungesättigtes cyclisches Carbonsäureanhydrid zugeführt wird, beispielsweise etwa 1 Mol Carbonsäureanhydrid pro Mol vorhandener OH-Gruppen. Danach wird nochmals eine gewünschte Menge an Alkylenoxidverbindungen addiert um das Hydroxylgruppen tragende Präpolymer zu erhalten. Die vorgenannte Reaktionsfolge lässt sich auch ein- oder mehrfach wiederholen, so dass eine gewünschte Zahl, insbesondere mehr als eine Doppelbindung pro Zerewitinoff-aktivem H-Atom in das Präpolymer eingebaut werden kann. So können schließlich beispielsweise 2 oder mehr, insbesondere 3 oder mehr Aminfunktionalitäten pro Zerewitinoff-aktivem H-Atom durch Addition an die Doppelbindungen eingeführt werden. Natürlich lassen sich die Doppelbindungen in das Präpolymer auch durch parallele Dosierung von einem oder mehreren Alkylenoxidverbindungen und einem oder mehreren ungesättigten cyclischen Carbonsäureanhydriden zu der einen oder mehreren Zerewitinoff-aktive H-Atome tragenden Starterverbindungen einführen. Die Verteilung der Doppelbindungen auf die Polymerketten des Präpolymers erfolgt dann nach statistischen Gesetzmäßigkeiten, insbesondere werden die Blöcke der auf Alkylenoxidbausteinen basierenden Polyetherketten einer breiteren Längenverteilung unterliegen.

Im Rahmen der vorliegenden Erfindung ist vorgesehen, dass die H-funktionelle Starterverbindung wenigstens ein Zerewitinoff-aktives H-Atom trägt. Unter einem Zerewitinoff-aktiven H-Atom wird im Rahmen der vorliegenden Erfindung ein azides H-Atom oder "aktives" H-Atom verstanden. Ein solches kann in an sich bekannter Weise durch eine Reaktivität mit einem entsprechenden Grignard-Reagenz identifiziert werden. Die Menge an Zerewitinoff-aktiven H-Atomen wird typischerweise über die Methanfreisetzung gemessen, die bei einer Reaktion der zu überprüfenden Sub-

stanz mit Methylmagnesiumbormid ($CH_3$-MgBr) gemäß der folgenden Reaktionsgleichung frei wird:

$$CH_3\text{-MgBr} + ROH \rightarrow CH_4 + Mg(OR)Br$$

Zerewitinoff-aktive H-Atome stammen typischerweise von C-H aziden organischen Gruppen, - OH, -SH, $-NH_2$ oder -NHR mit R als organischem Rest sowie -COOH.

Besonders geeignete H-funktionelle Starterverbindungen besitzen eine H-Funktionalität von 1 bis 35, insbesondere 1 bis 16, bevorzugt 1 bis 8, wobei sich die H-Funktionalität auf die vorgenannten Zerewitinoff-aktiven H-Atome bezieht.

Die Molekulargewichte der H-funktionellen Starterverbindungen können über weite Bereiche variieren, wobei ein mittleres Molekulargewicht von 17 bis 1200 g/mol besonders bevorzugt ist, insbesondere von 62 bis 1000.

Neben den bevorzugt zu verwendenden hydroxyfunktionellen Startern können auch aminofunktionelle Starter eingesetzt werden.

Beispiele für hydroxyfunktionelle Starterverbindungen sind Methanol, Ethanol, 1-Propanol, 2-Propanol und höhere aliphatische Monole, insbesondere Fettalkohole, Phenol, alkylsubstituierte Phenole, Propylenglykol, Ethylenglykol, Diethylenglykol, Dipropylenglykol, 1,2-Butandiol, 1,3-Butandiol, 1,4-Butandiol, Hexandiol, Pentandiol, 3-Methyl-1,5-pentandiol, 1,12-Dodecandiol, Glycerin, Trimethylolpropan, Pentaerythrit, Sorbit, Saccharose, Hydrochinon, Brenzcatechin, Resorcin, Bisphenol F, Bisphenol A, 1,3,5-Trihydroxybenzol, sowie methylolgruppenhaltige Kondensate aus Formaldehyd und Phenol oder Harnstoff. Es können auch hochfunktionelle Starterverbindungen auf Basis von hydrierten Stärkehydrolyseprodukten eingesetzt werden. Solche sind beispielsweise in EP 1525244 A1 beschrieben.

Beispiele für aminogruppenhaltige H-funktionelle Starterverbindungen sind Ammoniak, Ethanolamin, Diethanolamin, Triethanolamin, Isopropanolamin, Diisopropanolamin, Ethylendiamin, Hexamethylendiamin, Anilin, die Isomere des Toluidins, die Isomere des Diaminotoluols, die Isomere des Diaminodiphenylmethans sowie bei der Kondensation von Anilin mit Formaldehyd zu Diaminodiphenylmethan anfallende höherkernige Produkte, ferner methylolgruppenhaltige Kondensate aus Formaldehyd und Melamin sowie Mannichbasen.

Außerdem können als Starterverbindungen auch Ringöffnungsprodukte aus cyclischen Carbonsäureanhydriden und Polyolen eingesetzt werden. Beispiele sind Ringöffnungsprodukte aus Phthalsäureanhydrid oder Bernsteinsäureanhydrid einerseits und Ethylenglykol, Diethylenglykol, 1,2-Butandiol, 1,3-Butandiol, 1,4-Butandiol, Hexandiol, Pentandiol, 3-Methyl-1,5-pentandiol, 1,12-Dodecandiol, Glycerin, Trimethylolpropan, Pentaerythrit oder Sorbit andererseits. Daneben ist es auch möglich, ein- oder mehrfunktionelle Carbonsäuren direkt als Starterverbindungen einzusetzen.

Ferner können in dem Prozess auch vorgefertigte Alkylenoxidadditionsprodukte der erwähnten Starterverbindungen, also Polyetherpolyole vorzugsweise mit OH-Zahlen von 160 bis 1000 mg KOH/g, bevorzugt 250 bis 1000 mg KOH/g, als Starterverbindungen eingesetzt, bzw. dem Reaktionsgemisch zugesetzt werden. Auch ist es möglich, im erfindungsgemäßen Prozess Polyesterpolyole vorzugsweise mit OH-Zahlen im Bereich von 6 bis 800 mg KOH/g als Co-Starter einzusetzen. Hierfür geeignete Polyesterpolyole können beispielsweise aus organischen Dicarbonsäuren mit 2 bis 12 Kohlenstoffatomen und mehrwertigen Alkoholen, vorzugsweise Diolen, mit 2 bis 12 Kohlenstoffatomen, vorzugsweise 2 bis 6 Kohlenstoffatomen nach bekannten Verfahren hergestellt werden.

Des Weiteren können als H-funktionelle Startersubstanzen Polycarbonatpolyole, Polyestercarbonatpolyole oder Polyethercarbonatpolyole, bevorzugt Polycarbonatdiole, Polyestercarbonatdiole oder Polyethercarbonatdiole vorzugsweise jeweils mit OH-Zahlen im Bereich von 6 bis 800 mg KOH/g, als Starter oder Co-Starter verwendet werden. Diese werden beispielsweise durch Umsetzung von Phosgen, Dimethylcarbonat, Diethylcarbonat oder Diphenylcarbonat mit di- oder höherfunktionellen Alkoholen oder Polyesterpolyolen oder Polyetherpolyolen hergestellt.

In Schritt a) des erfindungsgemäßen Verfahrens dienen bevorzugt aminogruppenfreie H-funktionelle Starterverbindungen mit Hydroxygruppen als Träger der aktiven Wasserstoffe, wie beispielsweise Methanol, Ethanol, 1-Propanol, 2-Propanol und höhere aliphatische Monole, insbesondere Fettalkohole, Phenol, alkylsubstituierte Phenole, Propylenglykol, Ethylenglykol, Diethylenglykol, Dipropylenglykol, 1,2-Butandiol, 1,3-Butandiol, 1,4-Butandiol, Hexandiol, Pentandiol, 3-Methyl-1,5-pentandiol, 1,12-Dodecandiol, Glycerin, Trimethylolpropan, Pentaerythrit, Sorbit, Saccharose, Hydrochinon, Brenzcatechin, Resorcin, Bisphenol F, Bisphenol A, 1,3,5-Trihydroxybenzol, methylolgruppenhaltige Kondensate aus Formaldehyd und Phenol und hydrierte Stärkehydrolyseprodukte. Es können auch Gemische verschiedener H-funktioneller Starterverbindungen eingesetzt werden. Ist im Folgenden von einer H-funktionellen Starterverbindung die Rede, so sind damit grundsätzlich auch Gemische H-funktioneller Starterverbindungen gemeint, sofern dies nicht ausdrücklich ausgeschlossen wird.

Für das im Rahmen des erfindungsgemäßen Verfahrens eingesetzte ungesättigte zyklische Carbonsäureanhydrid kommen sämtliche dem Fachmann als solche bekannten Verbindungen in Frage. Dies sind beispielsweise ungesättigte zyklische Dicarbonsäureanhydride wie Maleinsäureanhydrid, Tetrahydrophthalsäureanhydrid, insbesondere 3,4,5,6-Tetrahydrophthalsäureanhydrid sowie Kombinationen hiervon.

Werden mehrere ungesättigte cyclische Carbonsäureanhydride verwendet, so können diese ebenfalls einzeln, im Gemisch oder blockweise dosiert werden. Es ist außerdem möglich, das cyclische Carbonsäureanhydrid oder die

cyclischen Carbonsäureanhydride dem Reaktionsgemisch parallel mit dem / den Alkylenoxid(en) oder als separaten Block, ohne gleichzeitige Alkylenoxiddosierung, zuzuführen. Ist im Folgenden von einem ungesättigten cyclischen Carbonsäureanhydrid die Rede, so sind damit grundsätzlich auch Gemische ungesättigter cyclischer Carbonsäureanhydride gemeint, sofern dies nicht ausdrücklich ausgeschlossen wird. Als erfindungsgemäß verwendbare Alkylenoxidverbindung können solche Vertreter ausgewählt sein, die 2 bis 24 Kohlenstoffatome aufweisen, insbesondere 2 bis 12 Kohlenstoffatome, weiter bevorzugt 2 bis 6 Kohlenstoffatome, sowie die Kombination unterschiedlicher Alkylenoxidverbindungen der vorgenannten Art. Bei den Alkylenoxiden mit 2 bis 24 Kohlenstoffatomen handelt es sich beispielsweise um eine oder mehrere Verbindungen ausgewählt aus der Gruppe bestehend aus Ethylenoxid, Propylenoxid, 1-Butenoxid, 2,3-Butenoxid, 2-Methyl-1,2-propenoxid (Isobutenoxid), 1-Pentenoxid, 2,3-Pentenoxid, 2-Methyl-1,2-butenoxid, 3-Methyl-1,2-butenoxid, 1-Hexenoxid, 2,3-Hexenoxid, 3,4-Hexenoxid, 2-Methyl-1,2-pentenoxid, 4-Methyl-1,2-pentenoxid, 2-Ethyl-1,2-butenoxid, 1-Heptenoxid, 1-Octenoxid, 1-Nonenoxid, 1-Decenoxid, 1-Undecenoxid, 1-Dodecenoxid, 4-Methyl-1,2-pentenoxid, Butadienmonoxid, Isoprenmonoxid, Cyclopentenoxid, Cyclohexenoxid, Cycloheptenoxid, Cyclooctenoxid, Styroloxid, Methylstyroloxid, Pinenoxid, ein- oder mehrfach epoxidierte Fette als Mono-, Di- und Triglyceride, epoxidierte Fettsäuren, $C_1$-$C_{24}$-Ester von epoxidierten Fettsäuren, Epichlorhydrin, Glycidol, und Derivate des Glycidols wie beispielsweise Methylglycidylether, Ethylglycidylether, 2-Ethylhexylglycidylether, Allylglycidylether, Glycidylmethacrylat sowie epoxidfunktionelle Alkyloxysilane wie beispielsweise 3-Glycidyloxypropyltrimethoxysilan, 3 -Glycidyloxypropyltriethoxysilan, 3 -Glycidyloxypropyltripropoxysilan, 3-Glycidyloxypropyl-methyl-dimethoxysilan, 3-Glycidyloxypropyl-ethyldiethoxysilan und 3-Glycidyloxypropyltriisopropoxysilan.

Als Alkylenoxide für die Herstellung der Polyetheresterpolyole in Schritt a) werden bevorzugt Ethylenoxid und/oder Propylenoxid eingesetzt. Die Alkylenoxide können einzeln, im Gemisch oder blockweise dosiert werden. Ist im Folgenden von einem Alkylenoxid oder einer Alkylenoxidverbindung die Rede, so sind damit grundsätzlich auch Gemische von Alkylenoxiden oder Alkylenoxidverbindungen oder die blockweise Dosierung von unterschiedlichen Alkylenoxiden bzw. Alkylenoxidverbindungen gemeint, sofern nicht ausdrücklich spezifiziert. Vorzugsweise werden Ethylenoxid und/oder Propylenoxid verwendet. Besonders bevorzugt wird Ethylenoxid in Anteilen größer 50 Gew.%, ganz besonders bevorzugt in Anteilen von größer 60 Gew.%, bezogen auf die Gesamtmasse der zu dosierenden Alkylenoxide, eingesetzt. Bei dem erfindungsgemäßen Verfahren kann ferner vorgesehen sein, dass das Stoffmengenverhältnis zwischen Carbonsäureanhydrid und der Zahl der Zerewitinoff-aktiven H-Atome der Starterverbindung so ausgewählt ist, dass möglichst alle Zerewitinoff-aktiven H-Atome umgesetzt werden. Für diese stöchiometrische Umsetzung kann das Stoffmengenverhältnis zwischen dem Carbonsäureanhydrid und der Zahl der Zerewitinoff-aktiven H-Atome der H-funktionellen Starterverbindung etwa 1 : 1 bis 1,5 : 1 betragen, insbesondere 1 : 1 bis 1,2 : 1.

Nach einer besonders bevorzugten Ausführungsform des erfindungsgemäßen Verfahrens wird das Stoffmengenverhältnis zwischen der Alkylenoxidverbindung und dem Carbonsäureanhydrid auf wenigstens 1,1 : 1, vorzugsweise auf wenigstens 2 : 1 eingestellt, besonders bevorzugt wenigstens auf 2,5 : 1. Bei dieser Ausführungsform des erfindungsgemäßen Verfahrens ist es möglich, Hydroxy-Aminopolymere mit einem mittleren Abstand von mehr als sieben kovalenten Bindungslängen zwischen der Aminfunktionalität und der Hydroxylgruppe zu synthetisieren. Das erfindungsgemäße Verfahren ist im Übrigen nicht auf die Verwendung der vorgenannten Monomere bzw. Co-Monomere beschränkt. So ist es beispielsweise möglich, dass wenigstens ein weiteres Co-Monomer im Schritt a) umgesetzt wird, welches insbesondere ausgewählt ist aus Lactonen, Lactiden, gesättigten bzw. aromatischen cyclischen Carbonsäureanhydriden, cyclischen Carbonaten und/ oder Kohlendioxid. Auf diese Weise kann das Eigenschaftsprofil des erhaltenen Hydroxy-Aminopolymers weiter modifiziert werden, beispielsweise in Bezug auf seine Reaktivität gegenüber Isocyanatgruppen, seine Polarität sowie hinsichtlich anderer chemischer oder physikalischer Eigenschaften des Hydroxy-Aminopolymers bzw. dessen Umsetzungsprodukts mit einem Polyisocyanat.

Im Rahmen des erfindungsgemäßen Verfahrens ist u.a. vorgesehen, dass ein primäres Amin oder Ammoniak an die Doppelbindung des Hydroxylgruppen-tragenden Präpolymers addiert wird. Geeignete Amine sind beispielsweise Ammoniak, aliphatische, cycloaliphatische und/oder araliphatische Monoamine mit einer primären Aminogruppe wie beispielsweise Methylamin, Ethylamin, , 1-Aminopropan, 2-Aminopropan, 1-Aminobutan, 2-Aminobutan, Isobutylamin, 1-Aminohexan, 2-Ethyl-1-Aminohexan, Dodecylamin, Octadecyl-amin, Cyclohexylamin und Benzylamin; aliphatische, cycloaliphatische und/oder araliphatische Monoamine mit einer primären Aminogruppe und einer sekundären Aminogruppe, wobei die sekundäre Aminogruppe auch Teil eines Ringsystems sein kann, wie beispielsweise N-Methylethylendiamin, N-Methylpropylendiamin, N-(2-Aminoethyl)-piperazin und 3-Amino-1,2,4-triazol; aliphatische, cycloaliphatische und/ oder heterocyclische Diamine mit einer primären und einer tertiären Aminogruppe und ggf. einer sekundären Aminogruppe wie beispielsweise N,N-dimethylethylendiamin, N,N-dimethyl-1,3-diaminopropan, N,N-dimethyl-1,8-diaminooctan, N,N-dimethyl-1,4-diaminocyclohexan; um aliphatische Diamine mit zwei primären und mindestens einer sekundären Aminogruppe, wie z. B. Diethylentriamin, Triethylentetramin, Tetraethylenpentamin und Bis-(3-aminopropyl)-amin. Des Weiteren sind Amine, welche neben der primären Aminogruppe auch Hydroxygruppen enthalten, wie beispielsweise Ethanolamin oder Isopropanolamin, für das erfindungsgemäße Verfahren geeignet. Bevorzugt sind dabei primäre Amine ausgewählt aus der Gruppe bestehend aus Ethylamin, 1-Aminobutan, Dodecylamin, Cyclohexylamin, Benzylamin, N,N-dimethyl-1,3-diaminopropan, Ethanolamin und Isopropanolamin.

Ebenfalls geeignet sind (cyclo)aliphatische Diamine. Hierbei handelt es sich um Verbindungen mit zwei primären Aminogruppen mit der allgemeinen Formel $NH_2$-R-$NH_2$, in der R für einen aliphatischen oder cycloaliphatischen Rest mit 2 bis 21, vorzugsweise 2 bis 15 und besonders bevorzugt 2 bis 10 Kohlenstoffatomen steht. Beispielhaft zu nennen sind Ethylendiamin, 1,2- und 1,3-Propylendiamin, 1,4-Diaminobutan, 1,6-Diaminohexan, 2,2,4- und 2,4,4-Trimethyl-1,6-diaminohexan, 1,4-Diaminocyclohexan, 1,5-Diamino-2-methylpentan, 5-Amino-1-aminomethyl-1,3,3-trimethylcyclohexan (Isophorondiamin), Bis-(4-aminocyclohexyl)-methan, Bis-(4-amino-3-methylcyclohexyl)-methan, 1-Amino-1-methyl-3(4)-aminomethylcyclohexan, Bis-(4-Amino-3,5-diethylcyclohexyl)-methan, Bis-aminomethyl-hexahydro-4,7-methano-indan, 2,3-, 2,4- und 2,6-Diamino-1-methylcyclohexan bzw. Gemische dieser Diamine. Bevorzugt sind dabei (cyclo)aliphatische Diamine ausgewählt aus der Gruppe bestehend aus Ethylendiamin, 1,4 Diaminobutan, 1,6 Diaminohexan, 1,5-Diamino-2-methylpentan, Bis-(4-aminocyclohexyl)-methan und Bis-(4-amino-3-methylcyclohexyl)-methan.

Die genannten Mono- und Oligoamine können natürlich auch als Mischung eingesetzt werden. Ist im Folgenden von einem zu addierenden Amin die Rede, so sind damit grundsätzlich auch Gemische zu addierender Amine gemeint, sofern nicht ausdrücklich spezifiziert.

Das molare Verhältnis von primären Aminogruppen zu additionsfähigen Doppelbindungen beträgt vorzugsweise 0,01 : 1 bis 1,1 : 1, bevorzugt 0,1 : 1 bis 1,1 : 1, besonders bevorzugt 0,5 : 1 bis 1,1 : 1 und ganz besonders bevorzugt 1 : 1 bis 1,1 : 1. Die Reaktion kann katalysiert oder unkatalysiert durchgeführt werden. Geeignete Katalysatoren sind beispielsweise Kupferacetat, Zinnchlorid oder Essigsäure. Bevorzugt erfolgt die Addition der Amine ohne Katalysatorzusatz. Ein für diesen Schritt geeigneter Reaktionstemperaturbereich ist beispielsweise der von 0 °C bis 150 °C, bevorzugt von 10 °C bis 100 °C und besonders bevorzugt 20 °C bis 80 °C.

Bei dem erfindungsgemäßen Verfahren ist vorgesehen, dass im Schritt a), also bei der Umsetzung der H-funktionellen Starterverbindung mit dem ungesättigten zyklischen Carbonsäureanhydrid und/ oder der Addition der Alkylenoxidverbindung ein DMC-Katalysator eingesetzt wird. Dabei können auch Mischungen verschiedener DMC-Katalysatoren verwendet werden.

Geeignete DMC-Katalysatoren sind im Prinzip aus dem Stand der Technik bekannt und werden beispielsweise in US 3,404,109 A1, US 3,829,505 A1, US 3,941,849 A1 und US 5,158,922 A1 offenbart.

DMC-Katalysatoren, die z.B. in US 5,470,813 A1, EP 700949 A1, EP 743 093 A1, EP 761 708 A1, WO 97/40086 A1, WO 98/16310 A1 und WO 00/47649 A1 beschrieben sind, besitzen eine sehr hohe Aktivität in der Polymerisation von Alkylenoxiden und ggf. der Copolymerisation von Alkylenoxiden und ungesättigten cyclischen Carbonsäureanhydriden und ermöglichen die Herstellung von Polyetherpolyolen bei sehr geringen Katalysatorkonzentrationen (25 ppm oder weniger), so dass eine Abtrennung des Katalysators aus dem fertigen Produkt im Allgemeinen nicht mehr erforderlich ist. Ein typisches Beispiel sind die in EP 700 949 A1 beschriebenen hochaktiven DMC-Katalysatoren, die neben einer Doppelmetallcyanid-Verbindung wie Zinkhexacyanocobaltat(III) und einem organischen Komplexliganden wie tert.-Butanol noch einen Polyether mit einem zahlenmittlerem Molekulargewicht größer als 500 g/mol enthalten. Es ist auch möglich, die in EP Anmeldenummer 10163170.3 offenbarten alkalischen DMC-Katalysatoren einzusetzen.

Zur Herstellung der Doppelmetallcyanid-Verbindungen geeignete cyanidfreie Metallsalze besitzen bevorzugt die allgemeine Formel (I),

$$M(X)_n \qquad (I)$$

wobei

M ausgewählt ist aus den Metallkationen $Zn^{2+}$, $Fe^{2+}$, $Ni^{2+}$, $Mn^{2+}$, $Co^{2+}$, $Sr^{2+}$, $Sn^{2+}$, $Pb^{2+}$ und, $Cu^{2+}$, bevorzugt ist M $Zn^{2+}$, $Fe^{2+}$, $Co^{2+}$ oder $Ni^{2+}$,
X für ein oder mehrere (d.h.verschiedene) Anionen steht, welches vorzugsweise ausgewählt ist aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;
n ist 1, wenn X = Sulfat, Carbonat oder Oxalat ist und
n ist 2, wenn X = Halogenid, Hydroxid, Cyanat, Thiocyanat, Isocyanat, Isothiocyanat oder Nitrat ist.

Weiter geeignete cyanidfreie Metallsalze besitzen die allgemeine Formel (II),

$$M_r(X)_3 \qquad (II)$$

wobei

M ausgewählt ist aus den Metallkationen $Fe^{3+}$, $Al^{3+}$ und $Cr^{3+}$,
X steht für einen oder unterschiedliche Anionentypen, wobei das Anion vorzugsweise ausgewählt ist aus der Gruppe der Halogenide (d.h. Fluorid, Chlorid, Bromid, Iodid), Hydroxid, Sulfat, Carbonat, Cyanat, Thiocyanat, Isocyanat,

Isothiocyanat, Carboxylat, Oxalat und Nitrat;
r ist 2, wenn X = Sulfat, Carbonat oder Oxalate ist und
r ist 1, wenn X = Halogenid, Hydroxid, Cyanat, Thiocyanat, Isocyanat, Isothiocyanat, Carboxylat oder Nitrat ist.

Andere geeignete cyanidfreie Metallsalze besitzen die allgemeine Formel (III),

$$M(X)_s \qquad (III)$$

wobei

M ausgewählt ist aus den Metallkationen $Mo^{4+}$, $V^{4+}$ und $W^{4+}$
X steht für einen oder unterschiedliche Anionentypen, wobei das Anion vorzugsweise ausgewählt ist aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;
s ist 2, wenn X = Sulfat, Carbonat oder Oxalat ist und
s ist 4, wenn X = Halogenid, Hydroxid, Cyanat, Thiocyanat, Isocyanat, Isothiocyanat, Carboxylat oder Nitrat ist.

Ebenfalls geeignete cyanidfreie Metallsalze besitzen die allgemeine Formel (IV),

$$M(X)_t \qquad (IV)$$

wobei

M ausgewählt ist aus den Metallkationen $Mo^{6+}$ und $W^{6+}$
X steht für einen oder unterschiedliche Anionentypen, wobei das Anion vorzugsweise ausgewählt aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;
t ist 3, wenn X = Sulfat, Carbonat oder Oxalat ist und
t ist 6, wenn X = Halogenid, Hydroxid, Cyanat, Thiocyanat, Isocyanat, Isothiocyanat, Carboxylat oder Nitrat ist,

Beispiele geeigneter cyanidfreier Metallsalze sind Zinkchlorid, Zinkbromid, Zinkjodid, Zinkacetat, Zinkacetylacetonat, Zinkbenzoat, Zinknitrat, Eisen(II)sulfat, Eisen(II)bromid, Eisen-(II)chlorid, Cobalt(II)chlorid, Cobalt(II)thiocyanat, Nickel(II)chlorid und Nickel(II)nitrat. Es können auch Mischungen verschiedener Metallsalze eingesetzt werden.
Zur Herstellung der Doppelmetallcyanid-Verbindungen geeignete Metallcyanidsalze besitzen bevorzugt die allgemeine Formel (V)

$$(Y)_aM'(CN)_b(A)_c \qquad (V)$$

wobei

M' ausgewählt ist aus einem oder mehreren Metallkationen der Gruppe bestehend aus Fe(II), Fe(III), Co(II), Co(III), Cr(II), Cr(III), Mn(II), Mn(III), Ir(III), Ni(II), Rh(III), Ru(II), V(IV) und V(V), bevorzugt ist M' ein oder mehrere Metallkationen der Gruppe bestehend aus Co(II), Co(III), Fe(II), Fe(III), Cr(III), Ir(III) und Ni(II),
Y ausgewählt ist aus einem oder mehreren Metallkationen der Gruppe bestehend aus Alkalimetall (d.h. $Li^+$, $Na^+$, $K^+$, $Rb^+$, $Cs^+$) und Erdalkalimetall (d.h. $Be^{2+}$, $Ca^{2+}$, $Mg^{2+}$, $Sr^{2+}$, $Ba^{2+}$),
A ausgewählt ist aus einem oder mehreren Anionen der Gruppe bestehend aus Halogeniden (d..h. Fluorid, Chlorid, Bromid, Iodid), Hydroxid, Sulfat, Carbonat, Cyanat, Thiocyanat, Isocyanat, Isothiocyanat, Carboxylat, Oxalat oder Nitrat und
a, b und c sind ganzzahlige Zahlen, wobei die Werte für a, b und c so gewählt sind, dass die Elektroneutralität des Metallcyanidsalzes gegeben ist; a ist vorzugsweise 1, 2, 3 oder 4; b ist vorzugsweise 4, 5 oder 6; c besitzt bevorzugt den Wert 0.

Beispiele geeigneter Metallcyanidsalze sind Kaliumhexacyanocobaltat(III), Kaliumhexacyanoferrat(II), Kaliumhexacyanoferrat(III), Calciumhexacyanocobaltat(III) und Lithiumhexacyanocobaltat(III).
Bevorzugte Doppelmetallcyanid-Verbindungen, die in den erfindungsgemäßen DMC-Katalysatoren enthalten sind, sind Verbindungen der allgemeinen Formel (VI)

$$M_x[M'_{x'}(CN)_y]_z \qquad (VI),$$

worin M wie in Formel (I) bis (IV) und
M' wie in Formel (V) definiert ist, und
x, x', y und z sind ganzzahlig und so gewählt, dass die Elektronenneutralität der Doppelmetallcyanidverbindung gegeben ist.
Vorzugsweise ist
x = 3, x' = 1, y = 6 und z = 2,
M = Zn(II), Fe(II), Co(II) oder Ni(II) und
M' = Co(III), Fe(III), Cr(III) oder Ir(III).

Beispiele bevorzugt eingesetzter Doppelmetallcyanidverbindungen sind Zinkhexacyanocobaltat(III), Zinkhexacyanoiridat(III), Zinkhexacyanoferrat(III) und Cobalt(II)hexacyanocobaltat(III). Weitere Beispiele geeigneter Doppelmetallcyanid-Verbindungen sind z.B. US 5,158,922 A1 zu entnehmen. Besonders bevorzugt verwendet wird Zinkhexacyanocobaltat(III). Die bei der Herstellung der DMC-Katalysatoren zugesetzten organischen Komplexliganden sind beispielsweise in US-A 5,158,922 A1, US 3,404,109 A1, US 3,829,505 A1, US 3,941,849 A1, EP 700949 A1, EP 761708 A1, JP 4145123 A1, US 5,470,813 A1, EP 743 093 A1 und WO 97/40086 A1 offenbart. Beispielsweise werden als organische Komplexliganden wasserlösliche, organische Verbindungen mit Heteroatomen, wie Sauerstoff, Stickstoff, Phosphor oder Schwefel, die mit der Doppelmetallcyanid - Verbindung Komplexe bilden können, eingesetzt. Bevorzugte organische Komplexliganden sind Alkohole, Aldehyde, Ketone, Ether, Ester, Amide, Harnstoffe, Nitrile, Sulfide und deren Mischungen. Besonders bevorzugte organische Komplexliganden sind aliphatische Ether (wie Dimethoxyethan), wasserlösliche aliphatische Alkohole (wie Ethanol, Isopropanol, n-Butanol, iso-Butanol, sek.-Butanol, tert-Butanol, 2-Methyl-3-buten-2-ol und 2-Methyl-3-butin-2-ol), Verbindungen, die sowohl aliphatische oder cycloaliphatische Ethergruppen wie auch aliphatische Hydroxylgruppen enthalten (wie z.B. Ethylenglykol-mono-tert.-butylether, Diethylenglykol-mono-tert.-butylether, Tripropylenglykol-mono-methylether und 3-Methyl-3-oxetan-methanol). Höchst bevorzugte organische Komplexliganden sind ausgewählt aus einer oder mehrerer Verbindungen der Gruppe bestehend aus Dimethoxyethan, tert-Butanol, 2-Methyl-3-buten-2-ol, 2-Methyl-3-butin-2-ol, Ethylenglykol-mono-tert.-butylether und 3-Methyl-3-oxetan-methanol.

Optional werden bei der Herstellung der erfindungsgemäß bevorzugten DMC-Katalysatoren eine oder mehrere komplexbildende Komponente(n) aus den Verbindungsklassen der Polyether, Polyester, Polycarbonate, Polyalkylenglykolsorbitanester, Polyalkylenglykolglycidylether, Polyacrylamid, Poly(acrylamid-co-acrylsäure), Polyacrylsäure, Poly(acrylsäure-co-maleinsäure), Polyacrylnitril, Polyalkylacrylate, Polyalkylmethacrylate, Polyvinylmethylether, Polyvinylethylether, Polyvinylacetat, Polyvinylalkohol, Poly-N-vinylpyrrolidon, Poly(N-vinylpyrrolidon-co-acrylsäure), Polyvinylmethylketon, Poly(4-vinylphenol), Poly(acrylsäure-co-styrol), Oxazolinpolymere, Polyalkylenimine, Maleinsäure- und Maleinsäureanhydridcopolymere, Hydroxyethylcellulose und Polyacetale, oder der Glycidylether, Glycoside, Carbonsäureester mehrwertiger Alkohole, Gallensäuren oder deren Salze, Ester oder Amide, Cyclodextrine, Phosphorverbindungen, $\alpha,\beta$-ungesättigten Carbonsäureester oder ionische oberflächen- bzw. grenzflächenaktiven Verbindungen eingesetzt.

Bevorzugt werden bei der Herstellung der erfindungsgemäß bevorzugten DMC-Katalysatoren im ersten Schritt die wässrigen Lösungen des Metallsalzes (z.B. Zinkchlorid), eingesetzt im stöchiometrischen Überschuss (mindestens 50 Mol-%) bezogen auf Metallcyanidsalz, (also mindestens ein molares Verhältnis von cyanidfreiem Metallsalz zu Metallcyanidsalz von 2,25 zu 1,00) und des Metallcyanidsalzes (z.B. Kaliumhexacyanocobaltat) in Gegenwart des organischen Komplexliganden (z.B. tert.-Butanol) umgesetzt, so dass sich eine Suspension bildet, die die Doppelmetallcyanid-Verbindung (z.B. Zinkhexacyanocobaltat), Wasser, überschüssiges cyanidfreies Metallsalz, und den organischen Komplexliganden enthält. Der organische Komplexligand kann dabei in der wässrigen Lösung des cyanidfreien Metallsalzes und/oder des Metallcyanidsalzes vorhanden sein, oder er wird der nach Ausfällung der Doppelmetallcyanid-Verbindung erhaltenen Suspension unmittelbar zugegeben. Es hat sich als vorteilhaft erwiesen, die wässrigen Lösungen des cyanidfreien Metallsalzes und des Metallcyanidsalzes und den organischen Komplexliganden unter starkem Rühren zu vermischen. Optional wird die im ersten Schritt gebildete Suspension anschließend mit einer weiteren komplexbildenden Komponente behandelt. Die komplexbildende Komponente wird dabei bevorzugt in einer Mischung mit Wasser und organischem Komplexliganden eingesetzt. Ein bevorzugtes Verfahren zur Durchführung des ersten Schrittes (d.h. der Herstellung der Suspension) erfolgt unter Einsatz einer Mischdüse, besonders bevorzugt unter Einsatz eines Strahldispergators wie in WO 01/39883 A1 beschrieben.

Im zweiten Schritt erfolgt die Isolierung des Feststoffs (d.h. die Vorstufe des erfindungsgemäßen Katalysators) aus der Suspension durch bekannte Techniken, wie Zentrifugation oder Filtration. In einer bevorzugten Ausführungsvariante zur Herstellung des Katalysators wird der isolierte Feststoff anschließend in einem dritten Verfahrensschritt mit einer wässrigen Lösung des organischen Komplexliganden gewaschen (z.B. durch Resuspendieren und anschließende erneute Isolierung durch Filtration oder Zentrifugation). Auf diese Weise können zum Beispiel wasserlösliche Nebenprodukte, wie Kaliumchlorid, aus dem erfindungsgemäßen Katalysator entfernt werden. Bevorzugt liegt die Menge des organischen Komplexliganden in der wässrigen Waschlösung zwischen 40 und 80 Gew.-%, bezogen auf die Gesamtlösung.

Optional wird im dritten Schritt der wässrigen Waschlösung eine weitere komplexbildende Komponente, bevorzugt im Bereich zwischen 0,5 und 5 Gew.-%, bezogen auf die Gesamtlösung, zugefügt.

Außerdem ist es vorteilhaft, den isolierten Feststoff mehr als einmal zu waschen. Hierzu kann z.B. der erste Waschvorgang wiederholt werden. Bevorzugt ist es aber, für weitere Waschvorgänge nicht wässrige Lösungen zu verwenden, z.B. eine Mischung aus organischem Komplexliganden und weiterer komplexbildender Komponente.

Der isolierte und gegebenenfalls gewaschene Feststoff wird anschließend, gegebenenfalls nach Pulverisierung, bei Temperaturen von im allgemeinen 20 - 100 °C und bei Drücken von im allgemeinen 0,1 mbar bis Normaldruck (1013 mbar) getrocknet.

Ein bevorzugtes Verfahren zur Isolierung der erfindungsgemäßen DMC-Katalysatoren aus der Suspension durch Filtration, Filterkuchenwäsche und Trocknung wird in WO 01/80994 A1 beschrieben.

Die Konzentration an in Schritt a) eingesetztem DMC-Katalysator beträgt 5 bis 1000 ppm, bevorzugt 10 bis 900 ppm und besonders bevorzugt 20 bis 800 ppm, bezogen auf die Menge des herzustellenden Hydroxylgruppen tragenden Präpolymers. Je nach Anforderungsprofil der der Aminaddition nachgeschalteten Anwendung kann der DMC-Katalysator im Produkt belassen oder (teilweise) abgetrennt werden Die (teilweise) Abtrennung des DMC-Katalysators kann beispielsweise durch Behandlung mit Adsorbentien erfolgen. Verfahren zur Abtrennung von DMC-Katalysatoren sind beispielsweise beschrieben in US 4,987,271 A1, DE 313 22 58 A1, EP 406 440 A1, US 5,391,722 A1, US 5,099,075 A1, US 4,721,818 A1, US 4,877,906 A1 und EP 385 619 A1.

Der H-funktionellen Verbindung können ggf. vor Inkontaktbringen mit dem DMC-Katalysator geringe Mengen einer anorganischen Mineralsäure, bevorzugt Phosphorsäure, zugesetzt werden, um etwaige Basenspuren in der H-funktionellen Starterverbindung zu neutralisieren.

Wird das erfindungsgemäße Verfahren unter Verwendung von Doppelmetallcyanid-Katalysatoren durchgeführt, so ist es weiterhin von Vorteil, zunächst die H-funktionelle Starterverbindung und den Katalysator vorzulegen, eine Teilmenge der Alkylenoxid-Verbindung sowie gegebenenfalls weiterer Co-Monomere zuzudosieren und erst anschließend das ungesättigte zyklische Carbonsäureanhydrid zuzugeben. Auf diese Weise kann ausgehend von der H-funktionellen Starterverbindung zunächst ein doppelbindungsfreies Polymergerüst aufgebaut werden. Zu diesem Zweck können sämtliche der vorgenannten Alkylenoxidverbindungen bzw. ggf. zusätzliche Co-Monomere verwendet werden. Das ungesättigte zyklische Carbonsäureanhydrid wird typischerweise dann der Reaktionsmischung zugeführt, wenn die vorgenannte Additionsreaktion der Alkylenoxidverbindung an die H-funktionelle Starterverbindung abgeschlossen ist.

Nachdem das ungesättigte zyklische Carbonsäureanhydrid zugegeben wurde, wird anschließend nochmals die Alkylenoxidverbindung zugegeben sowie gegebenenfalls weiteres Co-Monomer. Hierbei kann durch Wahl der Stoffmengen der Alkylenoxidverbindung(en) in Bezug auf den Stoffmengengehalt des zugegebenen ungesättigten zyklischen Carbonsäureanhydrids der Abstand zwischen der Aminfunktionalität und der Hydroxylgruppe wie vorgenannt beschrieben eingestellt werden, wobei insbesondere mehr als 1 mol der Alkylenoxidverbindung pro mol Zerewitinoff-aktivem Wasserstoff zugegeben wird. Auch durch Zugabe weiteren Co-Monomers kann auf den Abstand dieser beiden Funktionalitäten voneinander eingewirkt werden. Wie bereits oben erwähnt, kann anschließend nochmals Carbonsäureanhydrid und nach dessen Abreaktion weitere Alkylenoxidverbindung hinzugegeben werden, um die Möglichkeit zum Einbau von mehr als einer Aminfunktion pro Zerewitinoff-aktivem H-Atom vorzusehen.

Im Folgenden wird Schritt a) des erfindungsgemäßen Verfahrens detailliert beschrieben, wobei die vorliegende Erfindung nicht auf die folgende Darstellung beschränkt ist:

In einer Ausführungsform des erfindungsgemäßen Verfahrens wird die H-funktionelle Verbindung mit dem DMC-Katalysator zunächst in einem Reaktor / Reaktorsystem vorgelegt. Der H-funktionellen Verbindung können ggf. vor Inkontaktbringen mit dem DMC-Katalysator geringe Mengen einer anorganischen Mineralsäure, bevorzugt Phosphorsäure, zugesetzt werden, um etwaige Basenspuren in der H-funktionellen Starterverbindung zu neutralisieren, bzw. um den Produktionsprozess generell stabiler zu gestalten.

Nach Aufheizen auf Temperaturen von 50 bis 160 °C, insbesondere 60 bis 140 °C, ganz besonders bevorzugt 70 bis 140 °C wird der Reaktorinhalt in einer bevorzugten Verfahrensvariante mit Inertgas über einen Zeitraum bevorzugt 10 bis 60 min. unter Rühren gestrippt. Beim Strippen mit Inertgas werden flüchtige Bestandteile unter Einleiten von Inertgasen in die Flüssigphase bei gleichzeitig angelegtem Vakuum, bei einem absoluten Druck von 5 bis 500 mbar, entfernt. Nach dem Eindosieren von typischerweise 5 bis 20 Gew.-% eines oder mehrerer Alkylenoxide, enthaltend ggf. bereits eine kleine Menge des ungesättigten cyclischen Carbonsäureanhydrids und / oder weiteren Co-Monomers, bezogen auf die Menge an vorgelegter H-funktioneller Verbindung, wird der DMC-Katalysator aktiviert.

Die Zugabe eines oder mehrerer Alkylenoxide und ggf. einer kleinen Menge des ungesättigten cyclischen Carbonsäureanhydrids und / oder weiteren Co-Monomers kann vor, während oder nach dem Aufheizen des Reaktorinhaltes auf Temperaturen von 50 bis 160 °C, bevorzugt 60 bis 140 °C, ganz besonders bevorzugt 70 bis 140 °C geschehen; sie erfolgt bevorzugt nach dem Strippen. Die Aktivierung des Katalysators macht sich durch einen beschleunigten Abfall

des Reaktordruckes bemerkbar, wodurch der beginnende Alkylenoxidumsatz / Umsatz des ungesättigten cyclischen Carbonsäurenahydrids angezeigt wird.

Dem Reaktionsgemisch kann sodann die gewünschte Menge Alkylenoxid bzw. Alkylenoxidgemisch, ggf. gemeinsam mit der zu dosierenden Menge an ungesättigtem zyklischen Carbonsäureanhydrid und / oder weiterem Co-Monomer kontinuierlich zugeführt werden, wobei eine Reaktionstemperatur von 20 bis 200 °C, bevorzugt aber von 50 bis 160 °C gewählt wird. Die Reaktionstemperatur ist in den vielen Fällen identisch mit der Aktivierungstemperatur. Dem Reaktionsgemisch oder der H-funktionellen Verbindung kann vor Zudosierung des ungesättigten zyklischen Carbonsäureanhydrids ggf. ein Inhibitor wie beispielsweise ein Phenolderivat, Vitamin E oder Phenothiazin zugesetzt werden.

Oft erfolgt die Katalysatoraktivierung bereits so schnell, dass die Dosierung einer separaten Menge Alkylenoxid / des ungesättigten cyclischen Carbonsäureanhydrids zur Katalysatoraktivierung entfallen kann und direkt, gegebenenfalls zunächst mit einer reduzierten Dosierrate, mit der kontinuierlichen Dosierung des Alkylenoxids und des ungesättigten cyclischen Carbonsäureanhydrids begonnen werden kann. Auch kann die Reaktionstemperatur während der Alkylenoxiddosierphase / der Dosierung des ungesättigtem cyclischen Carbonsäureanhydrids innerhalb der beschriebenen Grenzen variiert werden. Ebenfalls können die Alkylenoxide und das cyclische Carbonsäureanhydrid dem Reaktor auf unterschiedliche Weise zugeführt werden: Möglich ist eine Dosierung in die Gasphase oder direkt in die Flüssigphase, z. B. über ein Tauchrohr oder einen in der Nähe des Reaktorbodens in einer gut durchmischten Zone befindlichen Verteilerring.

Bei DMC-katalysierten Prozessen ist die Dosierung in die Flüssigphase die bevorzugte Variante. Das Alkylenoxid und das ungesättigte cyclische Carbonsäureanhydrid sollten dem Reaktor kontinuierlich derart zugeführt werden, dass die sicherheitstechnischen Druckgrenzen des verwendeten Reaktorsystems nicht überschritten werden. Insbesondere bei der Codosierung von ethylenoxidhaltigen Alkylenoxidgemischen oder reinem Ethylenoxid ist darauf zu achten, dass ein ausreichender Inertgaspartialdruck im Reaktor während der Anfahr- und Dosierphase aufrechterhalten wird. Dieser kann beispielsweise durch Edelgase oder Stickstoff eingestellt werden.

Bei Dosierung in die Flüssigphase sollten die Dosieraggregate selbstleerend ausgelegt sein, beispielsweise durch Anbringen der Dosierbohrungen an der Unterseite des Verteilerrings. Generell sollte durch apparative Maßnahmen, beispielsweise durch die Montage von Rückschlagklappen, ein Rückströmen von Reaktionsmedium in die Dosieraggregate und Eduktvorlagen verhindert werden. Wird ein Alkylenoxid- / Carbonsäureanhydridgemisch dosiert, können die jeweiligen Alkylenoxide und die jeweiligen ungesättigten cyclischen Carbonsäureanhydride dem Reaktor separat oder als Mischung zugeführt werden. Eine Vorvermischung der Alkylenoxide untereinander und mit dem ungesättigten cyclischen Carbonsäureanhydrid kann beispielsweise durch ein in der gemeinsamen Dosierstrecke befindliches Mischaggregat erreicht werden ("inline-blending"). Es hat sich auch bewährt, Alkylenoxide und ggf. das ungesättigte cyclische Carbonsäureanhydrid pumpendruckseitig in einen beispielsweise über Wärmetauscher geführten Umpumpkreislauf einzeln oder vorgemischt zu dosieren. Für die gute Durchmischung mit dem Reaktionsmedium ist es dann von Vorteil ein hochscherendes Mischaggregat in den Alkylenoxid-/ Carbonsäureanhydrid-/ Reaktionsmediumstrom zu integrieren. Die Temperatur der exothermen ringöffnenden Additionsreaktion wird durch Kühlung auf dem gewünschten Niveau gehalten. Gemäß dem Stand der Technik zur Auslegung von Polymerisationsreaktoren für exotherme Reaktionen (z.B. Ullmann's Encyclopedia of Industrial Chemistry, Vol. B4, pp 167ff, 5th Ed., 1992) erfolgt eine solche Kühlung im Allgemeinen über die Reaktorwand (z.B. Doppelmantel, Halbrohrschlange) sowie mittels weiterer intern im Reaktor und/oder extern im Umpumpkreislauf angeordneter Wärmetauscherflächen, z.B. an Kühlschlangen, Kühlkerzen, Platten-Rohrbündel- oder Mischerwärmetauschern. Diese sollten so ausgelegt sein, dass auch zu Beginn der Dosierphase, d. h. bei kleinem Füllstand, effektiv gekühlt werden kann.

Generell sollte in allen Reaktionsphasen durch Auslegung und Einsatz handelsüblicher Rührorgane für eine gute Durchmischung des Reaktorinhaltes gesorgt werden, wobei hier insbesondere ein- oder mehrstufig angeordnete Rührer oder großflächig über die Füllhöhe wirkende Rührertypen geeignet sind (siehe z. B. Handbuch Apparate; Vulkan-Verlag Essen, 1. Aufl. (1990), S.188 - 208). Technisch besonders relevant ist hierbei eine im Mittel über den gesamten Reaktorinhalt eingetragene Mischenergie, die im Allgemeinen im Bereich von 0,2 bis 5 W/l liegt, mit entsprechend höheren lokalen Leistungseinträgen im Bereich der Rührorgane selbst und ggf. bei niedrigen Füllständen. Um eine optimale Rührwirkung zu erzielen, können im Reaktor gemäß allgemeinem Stand der Technik Kombinationen aus Stromstörern (z. B. Flach- oder Rohrstromstörer) und Kühlschlangen (oder Kühlkerzen) angeordnet werden, die sich auch über den Behälterboden erstrecken können. Die Rührleistung des Mischaggregates kann während der Dosierphase auch füllstandsabhängig variiert werden, um in kritischen Reaktionsphasen einen besonders hohen Energieeintrag zu gewährleisten. Beispielsweise kann es vorteilhaft sein, feststoffhaltige Dispersionen, die zu Reaktionsbeginn beispielsweise bei der Verwendung von Saccharose vorliegen können, besonders intensiv zu durchmischen.

Außerdem sollte insbesondere beim Einsatz fester H-funktioneller Starterverbindungen durch die Wahl des Rühraggregates sichergestellt werden, dass eine ausreichende Dispergierung des Feststoffes im Reaktionsgemisch gewährleistet ist. Bevorzugt werden hier bodengängige Rührstufen sowie besonders zur Suspendierung geeignete Rührorgane eingesetzt. Ferner sollte die Rührergeometrie zur Minderung des Aufschäumens von Reaktionsprodukten beitragen. Das Aufschäumen von Reaktionsgemischen kann beispielsweise nach Ende der Dosier- und Nachreaktionsphase beobachtet werden, wenn Restepoxide zusätzlich im Vakuum bei absoluten Drücken im Bereich von 1 bis 500 mbar entfernt werden. Für solche Fälle haben sich Rührorgane als geeignet herausgestellt, die eine kontinuierliche Durchmischung der Flüssigkeitsoberfläche erzielen. Je nach Anforderung weist die Rührwelle ein Bodenlager und gegebenenfalls weitere Stützlager im Behälter auf. Der Antrieb der Rührerwelle kann dabei von oben oder unten erfolgen (mit zentrischer oder exzentrischer Anordnung der Welle).

Alternativ ist es auch möglich, die notwendige Durchmischung ausschließlich über einen Wärmetauscher geführten Umpumpkreislauf zu erzielen oder diesen zusätzlich zum Rühraggregat als weitere Mischkomponente zu betreiben, wobei der Reaktorinhalt nach Bedarf (typischerweise 1 bis 50 mal pro Stunde) umgepumpt wird.

Für die Durchführung des erfindungsgemäßen Verfahrens sind die unterschiedlichsten Reaktortypen geeignet. Vorzugsweise werden zylinderförmige Behälter eingesetzt, welche ein Höhen-/Durchmesserverhältnis von 1:1 bis 10:1 besitzen. Als Reaktorböden kommen beispielsweise Kugel-, Klöpper-, Flach,- oder Konusböden in Frage.

Nach Ende der Dosierung des Alkylenoxids und des ungesättigten cyclischen Carbonsäureanhydrids sowie ggf. weiterer Co-monomere in Schritt a) kann sich eine Nachreaktionsphase anschließen, in der restliches Alkylenoxid / ungesättigtes zyklisches Carbonsäureanhydrid / weiteres Co-Monomer abreagiert. Das Ende dieser Nachreaktionsphase ist erreicht, wenn kein weiterer Druckabfall im Reaktionskessel feststellbar ist. Spuren unreagierter Alkylenoxide / ungesättigter zyklischer Carbonsäureanhydride können nach der Reaktionsphase gegebenenfalls im Vakuum bei einem absoluten Druck von 1 bis 500 mbar oder durch Strippen quantitativ entfernt werden. Durch Strippen werden flüchtige Bestandteile, wie beispielsweise (Rest-)Alkylenoxide, unter Einleiten von Inertgasen oder Wasserdampf in die Flüssigphase bei gleichzeitig angelegtem Vakuum (beispielsweise durch Durchleiten von Inertgas bei einem Absolutdruck von 5 bis 500 mbar) entfernt. Das Entfernen flüchtiger Bestandteile, wie beispielsweise nicht umgesetzter Epoxide, entweder im Vakuum oder durch Strippen, erfolgt bei Temperaturen von 20 bis 200 °C, bevorzugt bei 50 bis 160 °C und vorzugsweise unter Rühren. Solche Strippvorgänge können auch in sog. Strippkolonnen durchgeführt werden, in denen dem Produktstrom ein Inertgas- oder Wasserdampfstrom entgegengeleitet wird. Bevorzugt wird das Strippen mit Inertgasen in Abwesenheit von Wasserdampf durchgeführt. Nach Erreichen von Druckkonstanz bzw. nach Entfernen flüchtiger Bestandteile durch Vakuum und/oder Strippen kann das Produkt aus dem Reaktor abgelassen werden.

Bei Verfahrensvariante A) kann die Dosierung des cyclischen Carbonsäureanhydrids in Schritt a) auch so erfolgen, dass die Alkylenoxiddosierung / Dosierung weiterer Comonomere unterbrochen wird, und, ggf. nach einer Nachreaktionsphase, das ungesättigte cyclische Carbonsäureanhydrid dem Reaktor zugeführt wird und nach Zuführung der gewünschten Menge an ungesättigtem zyklischem Carbonsäureanhydrid die Alkylenoxiddosierung / Dosierung weiterer Comonomere wieder aufgenommen wird. Diese Vorgehensweise kann natürlich während eines Reaktionsansatzes auch mehrmals wiederholt werden. Besonders bevorzugt bei dieser Verfahrensweise ist, dass der abschließende Alkylenoxidblock eine Menge von größer 1 mol Alkylenoxid pro mol aktiver H-Atome aus den als Starterverbindungen eingesetzten H-funktionellen Verbindungen umfasst. Auch hier kann dem Reaktionsgemisch oder der H-funktionellen Verbindung vor Zudosierung des ungesättigten zyklischen Carbonsäureanhydrids ggf. ein Inhibitor wie beispielsweise ein Phenolderivat, Vitamin E oder Phenothiazin zugesetzt werden.

Es ist ebenso möglich, das Verhältnis der Dosiergeschwindigkeiten von Alkylenoxiddosierung und der Dosierung des ungesättigten cyclischen Carbonsäureanhydrids während der Zudosierphase, d. h. der gemeinsamen Dosierung dieser beiden Komponenten gegeneinander kontinuierlich oder stufenweise zu varriieren, indem beispielsweise das Verhältnis des Dosierstroms des ungesättigten cyclischen Carbonsäureanhydrids zu dem des Alkylenoxids / der Alkylenoxide Werte von 0:1 bis 1:0 annimmt.

Ein Charakteristikum von DMC-Katalysatoren ist ihre ausgeprägte Empfindlichkeit gegen hohe Konzentrationen an Hydroxylgruppen, welche beispielsweise durch große Mengen an Startern wie Ethylenglykol, Propylenglykol, Glycerin, Trimethylolpropan, Sorbitol oder Saccharose hervorgerufen werden, und polare Verunreinigungen des Reaktionsgemisches bzw. des Starters oder der Starter. Die DMC-Katalysatoren können dann während der Reaktionsinitiierungsphase nicht in die polymerisationsaktive Form überführt werden. Verunreinigungen können beispielsweise Wasser oder Verbindungen mit einer hohen Zahl in enger Nachbarschaft stehender Hydroxylgruppen wie Kohlenhydrate und Kohlenhydratderivate sein. Auch Substanzen mit in enger Nachbarschaft stehenden Carbonylgruppen bzw. zu Hydroxylgruppen benachbarten Carbonylgruppen wirken sich nachteilig auf die Katalysatoraktivität aus.

Um Starter mit hohen Konzentrationen an OH-Gruppen, bzw. Starter mit als Katalysatorgiften anzusehenden Verunreinigungen dennoch DMC-katalysierten Alkylenoxidadditionsreaktionen unterziehen zu können, sollte die Hydroxylgruppenkonzentration gesenkt bzw. die Katalysatorgifte unschädlich gemacht werden. Hierzu können aus diesen Starterverbindungen mittels basischer Katalyse zunächst Vorpolymerisate hergestellt werden, welche dann nach Aufarbeitung mittels DMC-Katalyse in die erwünschten Alkylenoxidadditionsprodukte hoher Molmasse überführt werden. Unter diese Vorpolymerisate fallen bespielsweise die oben erwähnten als Starter geeigneten "vorgefertigten Alkylenoxidadditionsprodukte". Nachteilig bei dieser Vorgehensweise ist, dass solche oft mittels basischer Katalyse erhaltenen Vorpolymerisate sehr sorgfältig aufgearbeitet werden müssen, um die Deaktivierung des DMC-Katalysators durch über die Vorpolymerisate eingeschleppte basische Katalysatorspuren auszuschließen.

Dieser Nachteil kann durch das sogenannte Verfahren der kontinuierlichen Starterdosierung überwunden werden. Hierbei werden kritische Starterverbindungen im Reaktor nicht vorgelegt, sondern neben den Alkylenoxiden dem Reaktor während der Reaktion kontinuierlich zugeführt. Als Startmedium für die Reaktion können in diesem Verfahren Vorpolymerisate vorgelegt werden, auch ist die Verwendung kleiner Mengen des herzustellenden Produktes selbst als Startmedium möglich. Die Notwendigkeit, für weitere Alkylenoxidadditionen geeignete Vorpolymerisate zunächst separat herstellen zu müssen, entfällt somit.

In Variante B) von Schritt a) des erfindungsgemäßen Verfahrens werden daher ein Starterpolyol und der DMC-Katalysator im Reaktorsystem vorgelegt und die H-funktionelle Verbindung kontinuierlich gemeinsam mit dem Alkylenoxid und dem ungesättigten zyklischen Carbonsäureanhydrid zugeführt. Als Starterpolyol in Schritt a) sind Alkylenoxidadditionsprodukte wie beispielsweise Polyetherpolyole, Polyesterpolyole, Polyetheresterpolyole, Polycarbonatpolyole, Polyestercarbonatpolyole, Polyethercarbonatpolyole jeweils beispielsweise mit OH-Zahlen im Bereich von 3 bis 1000 mg KOH/g, vorzugsweise von 3 bis 300 mg KOH/g, und / oder gemäß Schritt a) separat hergestelltes Zwischenprodukt, geeignet. Vorzugsweise wird gemäß Schritt a) separat hergestelltes Zwischenprodukt als Starterpolyol in Schritt a) eingesetzt. In einer weniger bevorzugten Variante dieser Ausführungsform B) ist es ebenfalls möglich, das Verhältnis der Dosiergeschwindigkeiten von Alkylenoxiddosierung und der Dosierung des ungesättigten cyclischen Carbonsäureanhydrids während der Zudosierphase der drei Komponenten gegeneinander kontinuierlich oder stufenweise zu varriieren, indem beispielsweise das Verhältnis des Dosierstroms des ungesättigten zyklischen Carbonsäureanhydrids zu dem des Alkylenoxids / der Epoxide Werte von 0:1 bis 1:0 annimmt. Diese Ausführungsform ist weniger bevorzugt, da nach ihr das Zwischenprodukt nach Schritt a) in weniger einheitlicher Form erhalten wird.

Vorzugsweise wird in Ausführungsform B) von Schritt a) die Dosierung der H-funktionellen Verbindung und die des Alkylenoxids sowie des ungesättigten zyklischen Carbonsäureanhydrids gleichzeitig beendet, oder die H-funktionelle Verbindung und eine erste Teilmenge an Alkylenoxid und eine erste Teilmenge des ungesättigten zyklischen Carbonsäureanhydrids werden zunächst gemeinsam zudosiert, und anschließend wird die zweite Teilmenge an Alkylenoxid und ungesättigtem zyklischen Carbonsäureanhydrid zudosiert, wobei die Summen der ersten und zweiten Teilmenge an Alkylenoxid und der ersten und zweiten Teilmenge an ungesättigtem cyclischen Carbonsäureanhydrid der Gesamtmenge an in Schritt a) eingesetzter Menge an einem oder mehreren Alkylenoxiden bzw. an einem oder mehreren ungesättigten zyklischen Carbonsäureanhydriden entsprechen. Die erste Teilmenge beträgt vorzugsweise 60 bis 98 Gew. -% und die zweite Teilmenge beträgt 40 bis 2 Gew.-% der insgesamt in Schritt a) zu dosierenden Menge an Alkylenoxid. Die erste Teilmenge beträgt vorzugsweise 0 bis 100 Gew. -% und die zweite Teilmenge beträgt 100 bis 0 Gew.-% der insgesamt in Schritt a) zu dosierenden Menge eines oder mehrerer ungesättigter cyclischer Carbonsäureanhydride.

Wird die Zusammensetzung der Alkylenoxide und / oder die Zusammensetzung / die Dosierrate des einen oder der mehreren ungesättigten zyklischen Carbonsäureanydride nach Ende der Dosierung der H-funktionellen Verbindung geändert, lassen sich auch nach Verfahrensvariante B) Produkte mit Multiblockstrukturen herstellen. Auch bei Verfahrensvariante B) ist es bevorzugt, dass die Dosierung des ungesättigten zyklischen Carbonsäureanhydrids vor der Alkylenoxiddosierung beendet wird, besonders bevorzugt dergestalt, dass dieser abschließende Alkylenoxidblock eine Menge von größer 1 mol Alkylenoxid pro mol aktiver H-Atome aus den als Starterverbindungen eingesetzten H-funktionellen Verbindungen umfasst. Nach Zudosierung der Reagenzien kann sich eine Nachreaktionsphase anschließen, in der der Verbrauch an Alkylenoxid / ungesättigtem zyklischen Carbonsäureanhydrid durch Überwachung des Drucks quantifiziert werden kann. Nach Erreichen von Druckkonstanz kann das Produkt, gegebenenfalls nach Anlegen von Vakuum oder durch Strippen zur Entfernung von nicht umgesetzen Alkylenoxiden, wie oben beschrieben, abgelassen werden.

In Variante C) von Schritt a) des erfindungsgemäßen Verfahrens können die Zwischenprodukte vollkontinuierlich hergestellt werden. Hierzu werden neben Alkylenoxid und der H-funktionellen Verbindung sowie dem ungesättigten cyclischen Carbonsäureanhydrid auch der DMC-Katalysator dem Reaktor bzw. einem Reaktorsystem unter Alkoxylierungsbedingungen kontinuierlich zugeführt und das Produkt kontinuierlich dem Reaktor bzw. dem Reaktorsystem nach einer vorwählbaren mittleren Verweilzeit entnommen. Bei Verfahrensvariante C) ist es bevorzugt, dass als Reaktorsystem eine Reaktorkaskade eigesetzt wird, bei der sich zwischen dem Nachreaktor und dem eigentlichen Reaktor ein dritter, kontinuierlich betriebener Reaktor befindet, in den ausschließlich das eine oder mehrere Alkylenoxide kontinuierlich dosiert werden. In einer besonders bevorzugten Ausführungsform der Verfahrensvariante C) umfasst dieser abschließende Alkylenoxidblock eine Menge von größer 1 mol Alkylenoxidpro mol aktiver H-Atome aus den als Starterverbindungen eingesetzten H-funktionellen Verbindungen.

Es können sich kontinuierliche Nachreaktionsschritte, beispielsweise in einer Reaktorkaskade oder in einem Rohrreaktor anschließen. Flüchtige Bestandteile können im Vakuum und/oder durch Strippen, wie oben beschrieben, entfernt werden

Die OH-Zahlen der nach dem DMC-katalysierten Additionsschritt a) erhaltenen ungesättigten Polyetheresterpolyole weisen bevorzugt Werte von 3 mg KOH/g bis 200 mg KOH/g, besonders bevorzugt von 10 bis 60 mg KOH/g, ganz besonders bevorzugt von 20 bis 50 mg KOH/g, auf. Die OH-Zahl kann z. B. titrimetrisch nach der Vorschrift der DIN 53240 oder spektroskopisch über NIR bestimmt werden.

Unter Äquivalentmolmasse ist die durch die Zahl der aktiven Wasserstoffatome geteilte Gesamtmasse des aktive Wasserstoffatome enthaltenden Materials zu verstehen. Im Falle von hydroxygruppenhaltigen Materialien steht sie in folgender Beziehung zur OH-Zahl:

$$\text{Äquivalentmolmasse} = 56100 / \text{OH-Zahl [mg KOH/g]}$$

Den nach Schritt a) des erfindungsgemäßen Verfahrens erhältlichen Zwischenprodukten können gegebenenfalls Alterungsschutzmittel wie z. B. Antioxidantien zugesetzt werden.

Im Folgenden wird Schritt b) des erfindungsgemäßen Verfahrens detailliert beschrieben. Auch diese Darstellung ist lediglich beispielhaft und nicht als die vorliegende Erfindung beschränkend zu verstehen:
Für Schritt b) wird ein geeignetes Amin bei Temperaturen von 0 °C bis 150 °C, bevorzugt 10 °C bis 100 °C und besonders bevorzugt 20 °C bis 80 °C mit den Produkten aus Schritt a) zur Reaktion gebracht. Das molare Verhältnis von primären Aminogruppen zu additionsfähigen Doppelbindungen beträgt beispielsweise etwa 1 : 1 bis 1,1 : 1. Zwar kann die Reaktion mit Kupferacetat, Zinnchlorid oder Essigsäure katalysiert werden, sie wird jedoch bevorzugt ohne Katalysatorzusatz durchgeführt.

Im Allgemeinen werden die Amine unter Inertgas dem vorgelegten Zwischenprodukt aus Schritt a) zugeführt und bei den genannten Temperaturen über einen Zeitraum von 1 h bis ca. 48 h gerührt. Eine Vorvermischung der Amine mit dem Zwischenprodukt aus Schritt a) ist ebenfalls möglich, beipielsweise über ein in der gemeinsamen Dosierstrecke befindliches Mischaggregat ("inline-blending").

Der Reaktionsfortschritt kann über gängige Methoden, wie beispielsweise online oder offline durchgeführte gaschromatographische Untersuchungen oder spektroskopische Methoden, wie beispielsweise NMR- oder IR-Spektroskopie quantifiziert werden. Spuren unreagierter Amine oder etwaige Aminüberschüsse können nach der Reaktionsphase gegebenenfalls im Vakuum bei einem absoluten Druck von 1 bis 500 mbar oder durch Strippen quantitativ entfernt werden.

Die Umsetzung der Komponente aus Schritt a) mit dem oder den Aminen in Schritt b) kann prinzipiell im gleichen Reaktor wie die Herstellung der Komponente nach Schritt a) erfolgen. Es ist jedoch bevorzugt, die Umsetzung nach Schritt b) in einem anderen Reaktor durchzuführen, da im Reaktor verbleibende Aminspuren die Durchführung des nächsten DMC-katalysierten Schrittes a) behindern können.

Es hat sich als vorteilhaft erwiesen, Polyole für Polyurethan(harnstoff-)anwendungen ganz generell stets unter Intertgasatmosphäre zu handhaben. Dies gilt insbesondere für konventionelle alkalische Polyetherpolyole, wie sie beispielsweise unter Alkalimetallhydroxidkatalyse vor Abtrennung des Katalysators anfallen oder für unter Aminkatalyse erhaltene Produkte. Auch für salzfreie, aufgearbeitete und stabilisierte oder für unter DMC-Katalyse hergestellte Zwischen- und Fertigprodukte werden Handhabung und Lagerung unter Sauerstoffausschluss empfohlen. Dasselbe gilt für die nach dem erfindungsgemäßen Verfahren erhältlichen Hydroxy-Aminopolymere und deren nach Schritt a) erhältlichen Vorstufen. Geeignete Inertgase sind beispielsweise Edelgase, Stickstoff oder Kohlendioxid, besonders geeignet sind Edelgase oder Stickstoff. Durch die Unterbindung von Sauerstoffzutritt lassen sich Produktverfärbungen weitestgehend vermeiden, dies gilt insbesondere bei erhöhten Temperaturen, die im Allgemeinen genutzt werden um durch Senkung der Viskosität die Handhabung der (Zwischen-)Produkte zu erleichtern. Darüber hinaus entstehen unter Intergasatmosphäre auch deutlich weniger Peroxidgruppen, die unter Spaltung der Etherbindungen zur Bildung weiterer niedermolekularer oxidativer Abbauprodukte wie beispielsweise Acetaldehyd, Methanol, Ameisensäure, Ameisensäureester, Aceton und Formaldehyd Anlass geben. Somit können der Gehalt an leichtflüchtigen organischen Verbindungen in den (Zwischen-)Produkten gesenkt und Geruchsbelästigungen, gesundheitliche Beeinträchtigungen sowie Qualitätsminderungen vermieden werden.

Die vorliegende Erfindung betrifft ferner ein Hydroxy-Aminopolymer, das nach dem erfindungsgemäßen Verfahren erhältlich ist, wobei das Stoffmengenverhältnis zwischen der Alkylenoxidverbindung und dem Carbonsäureanhydrid wenigstens 1,1 : 1, vorzugsweise wenigstens 2 : 1 beträgt, besonders bevorzugt wenigstens 2,5 : 1 und wobei das Hydroxy-Aminopolymer weiter bevorzugt keiner Reinigung unterzogen wird, und wobei das erfindungsgemäße Hydroxy-Aminopolymer eine Struktur gemäß der allgemeinen Formel (VII) besitzt,

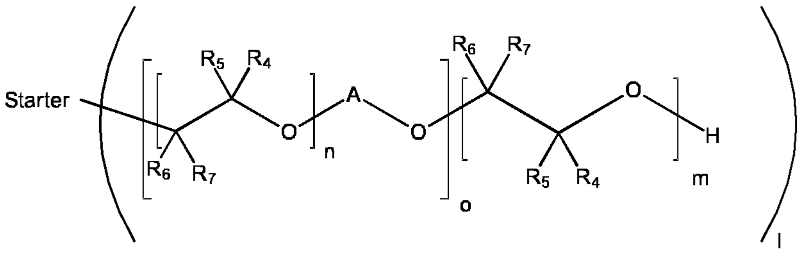

(VII)

wobei

"Starter" für das Radikal der H-funktionellen Starterverbindung steht,
A für eine Aspartatgruppe folgender Struktur der Formel (VIIIa) oder (VIIIb) steht,

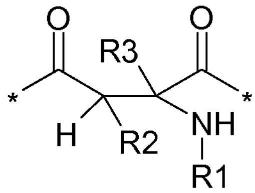

(VIIIa)

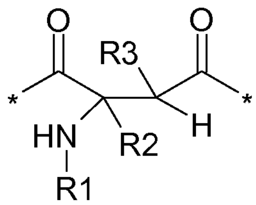

(VIIIb),

in der

| | |
|---|---|
| R2 und, R3 | unabhängig voneinander für Wasserstoff oder einen aliphatischen oder aromatischen Rest stehen und R2 und R3 auch Bestandteil eines cycloaliphatischen Ringsystems sein können, |
| R1 | für Wasserstoff oder einen aliphatischen, cycloaliphatischen oder aromatischen Rest steht, der auch Heteroatome, insbesondere Stickstoffatome oder Sauerstoffatome sowie Hydroxygruppen enthalten kann, |
| R4, R5, R6 und R7 | unabhängig voneinander für Wasserstoff oder einen aliphatischen oder aromatischen Rest stehen und R5 und R6 auch Bestandteil eines cycloaliphatischen Ringsystems sein können, |
| l für | die Zahl der Zerewitinoff-aktiven Wasserstoffatome der H-funktionellen Starterverbindung steht, |
| m, n und o | unabhängig voneinander und ganzzahlig sind, wobei n, o = 0 oder ≥ 1 und m ≥ 2 sind und n, m vorzugsweise 2 bis 430 betragen, insbesondere 3 bis 430, bevorzugt 4 bis 430 und o vorzugsweise 1 bis 100, insbesondere 1 bis 50 und bevorzugt 1 bis 10 beträgt, |

wobei die Äquivalentmolmasse der in der Formel VII gezeigten Struktur den Wert 18900 g/mol nicht übersteigt.

Bei der vorgenannten Struktur der Formel VII kann also die Laufziffer o unabhängig für jeden Ast der Verbindung gewählt sein.

Die vorliegende Erfindung ist zudem auf ein Polyurethanharnstoffpolymer gerichtet, welches durch Umsetzung eines Polyisocyanats mit einem Hydroxy-Aminopolymer, herstellbar nach dem erfindungsgemäßen Verfahren, erhältlich ist. Hierzu können die nach dem erfindungsgemäßen Verfahren erhältlichen Hydroxy-Aminopolymere alleine oder gegebenenfalls im Gemisch mit weiteren isocyanatreaktiven Komponenten mit organischen Polyisocyanaten, gegebenenfalls in Gegenwart von Treibmitteln, Katalysatoren und gegebenenfalls weiteren Zusatzstoffen wie z. B. Zellstabilisatoren zur

Reaktion gebracht werden und so als Komponenten von massiven oder geschäumten Polyurethanharnstoffen dienen. Folglich sind Polyurethanharnstoffe, bevorzugt massive oder geschäumte Polyurethanharnstoffe, insbesondere Beschichtungssysteme enthaltend die erfindungsgemäßen Hydroxy-Aminopolymere ebenfalls Gegenstand der Erfindung. Geeignete Polyisocyanate sind aliphatische, cycloaliphatische, araliphatische, aromatische und heterocyclische Polyisocyanate, wie sie z.B. von W. Siefken in Justus Liebigs Annalen der Chemie, 562, Seiten 75 bis 136, beschrieben werden, beispielsweise solche der Formel (IX)

$$Q(NCO)_n, \qquad (IX)$$

in der

n = 2 - 4, vorzugsweise 2 -3,
und
Q einen aliphatischen Kohlenwasserstoffrest mit 2 - 18, vorzugsweise 6 - 10 C-Atomen, einen cycloaliphatischen Kohlenwasserstoffrest mit 4 - 15, vorzugsweise 6 - 13 C-Atomen oder einen araliphatischen Kohlenwasserstoffrest mit 8 - 15, vorzugsweise 8 - 13 C-Atomen bedeuten.

Beispielsweise handelt es sich um solche Polyisocyanate, wie sie in der EP 0 007 502 A1, Seiten 7 - 8, beschrieben werden. Bevorzugt werden in der Regel die technisch leicht zugänglichen Polyisocyanate, z.B. das 2,4- und 2,6-Toluylendiisocyanat, sowie beliebige Gemische dieser Isomeren ("TDI"); Polyphenylpolymethylenpolyisocyanate, wie sie durch Anilin-Formaldehyd-Kondensation und anschließende Phosgenierung hergestellt werden ("rohes MDI") und Carbodiimidgruppen, Urethangruppen, Allophanatgruppen, Isocyanuratgruppen, Harnstoffgruppen oder Biuretgruppen aufweisenden Polyisocyanate ("modifizierte Polyisocyanate"), insbesondere solche modifizierten Polyisocyanate, die sich vom 2,4- und/oder 2,6-Toluylendiisocyanat bzw. vom 4,4'- und/oder 2,4'-Diphenylmethandiisocyanat ableiten. Die urethangruppenhaltigen Polyisocyanate (Präpolymere) können beispielsweise Reaktionsprodukte der Polyisocyanate mit Polyricinolsäureester-Polyolen oder aber beliebigen anderen Polyolen (beispielsweise konventionellen Polyolen) sein. Vorzugsweise wird als Polyisocyanat mindestens eine Verbindung ausgewählt aus der Gruppe bestehend aus 2,4- und 2,6-Toluylendiisocyanat, 4,4'- und 2,4'- und 2,2'-Diphenylmethandiisocyanat und Polyphenylpolymethylenpolyisocyanat ("Mehrkern-MDI") eingesetzt, besonders bevorzugt wird als Polyisocyanat eine Mischung enthaltend 4,4'-Diphenylmethandiisocyanat und 2,4'-Diphenylmethandiisocyanat und Polyphenylpolymethylenpolyisocyanat eingesetzt.

Neben den vorgenannten Polyisocyanaten können für die Herstellung der Polyurethan(harnstoff)polymere zusätzlich noch konventionelle Polyetherpolyole verwendet werden. Unter konventionellen Polyetherpolyolen im Sinne der Erfindung werden Verbindungen bezeichnet, die Alkylenoxidadditionsprodukte von Starterverbindungen mit zerewitinoff-aktiven Wasserstoffatomen, also Polyetherpolyole mit einer Hydroxylzahl gemäß DIN 53240 von ≥ 3 mg KOH/g bis ≤ 1000 mg KOH/g, vorzugweise von ≥ 5 mg KOH/g bis ≤ 600 mg KOH/g sind. Beispiele für solche Polyole sind dem Fachmann bekannt. Sie können eine Hydroxylzahl gemäß DIN 53240 von ≥ 3 mg KOH/g bis ≤ 1000 mg KOH/g, vorzugweise von ≥ 5 mg KOH/g bis ≤ 600 mg KOH/g aufweisen. Die für die Herstellung der konventionellen Polyetherpolyole eingesetzten Starterverbindungen mit Zerewitinoff-aktiven Wasserstoffatomen weisen meist Funktionalitäten von 2 bis 8 auf, bevorzugt von 3 bis 6, besonders bevorzugt von 3, und vorzugsweise sind die Starterverbindungen hydroxyfunktionell. Beispiele für hydroxyfunktionelle Starterverbindungen sind Propylenglykol, Ethylenglykol, Diethylenglykol, Dipropylenglykol, 1,2-Butandiol, 1,3-Butandiol, 1,4-Butandiol, Hexandiol, Pentandiol, 3-Methyl-1,5-pentandiol, 1,12-Dodecandiol, Glycerin, Trimethylolpropan, Triethanolamin, Pentaerythrit, Sorbitol, Saccharose, Hydrochinon, Brenzcatechin, Resorcin, Bisphenol F, Bisphenol A, 1,3,5-Trihydroxybenzol, methylolgruppenhaltige Kondensate aus Formaldehyd und Phenol oder Melamin oder Harnstoff. Vorzugsweise wird als Starterverbindung Glycerin und/oder Trimethylolpropan eingesetzt.

Geeignete Alkylenoxide für die konventionellen Polyetherpolyole sind beispielsweise Ethylenoxid, Propylenoxid, 1,2-Butylenoxid bzw. 2,3-Butylenoxid und Styroloxid. Bevorzugt werden Propylenoxid und Ethylenoxid dem Reaktionsgemisch einzeln, im Gemisch oder nacheinander zugeführt. Werden die Alkylenoxide nacheinander dosiert, so enthalten die hergestellten Produkte Polyetherketten mit Blockstrukturen. Produkte mit Ethylenoxidendblöcken sind beispielsweise durch erhöhte Konzentrationen an primären Endgruppen gekennzeichnet, welche den Systemen eine vorteilhafte Isocyanatreaktivität verleihen.

Schließlich ist die vorliegende Erfindung auf die Verwendung eines erfindungsgemäßen Hydroxy-Aminopolymers gerichtet zur Herstellung eines Polyurethanharnstoffpolymers.

Die vorliegende Erfindung wird im Folgenden anhand von Ausführungsbeispielen näher erläutert.

## Mess- und Bestimmungsmethoden:

### OH-Zahl, Säurezahl, Aminzahl und Viskosität

Die Bestimmung der OH-Zahlen erfolgte gemäß der Vorschrift der DIN 53240. Die Bestimmung der Säurezahlen erfolgte gemäß der Vorschrift der DIN EN ISO 2114. Die Bestimmung der Aminzahlen erfolgte gemäß der Vorschrift der DIN 53176. Die Viskositäten wurden mittels Rotationsviskosimeter (Physica MCR 51, Hersteller: Anton Paar) nach der Vorschrift der DIN 53018 ermittelt (Spindeltyp CC27, Scherratenbereich 16 - 128 $s^{-1}$).

### Molmassenverteilung

Die Molmassenverteilung wurde mittels Größenausschlusschromatographie (SEC) ermittelt. Verwendet wurde das Gerät Agilent 1100 Series der Fa. Agilent. Angegeben wird die Polydispersität PD für die Molekulargewichtsverteilung $M_w/M_n$, wobei $M_w$ für die gewichtsgemittelte Molmasse und $M_n$ für die zahlengemittelte Molmasse stehen. Weitere Angaben:

- Säulenkombination: 1 Vorsäule PSS, 5 µl, 8x50mm; 2 PSS SVD, 5 µl, 100 A°, 8x300mm; 2 PSS SVD, 5 µl, 1000 A°, 8x300mm, PSS ist der Hersteller der Säulen (Polymer Standard Solutions, Mainz)
- Auswertesoftware: WIN GPC der Fa. PSS
- Lösungsmittel: THF (Merck LiChrosolv)
- Flussrate: 1 ml / min
- Detektortyp: RI-Detektor (Brechungsindex), Shodex RI 74
- Verwendete Kalibrationsstandards: Kalibrierstandard der Fa. PSS auf Basis Polystyrol.

## Eingesetzte Rohstoffe

### Katalysator für die Alkylenoxid- / Säureanhydridaddition (DMC-Katalysator):

Doppelmetallcyanid-Katalysator, enthaltend Zinkhexacyanocobaltat, tert.-Butanol und Polypropylenglykol mit einem zahlenmittleren Molekulargewicht von 1000 g/mol; beschrieben in WO 01/80994 A1, Beispiel 6.

**Ambosol®:** Ausgefälltes, kolloidales synthetisch hergestelltes Magnesiumsilikat, bezogen von PQ Europe.

**Polyol A:** Polyether, erhalten durch Addition von Ethylenoxid an Propylenglykol unter KOH-Katalyse; OH-Zahl: 190 mg KOH / g, zahlenmittlere Molmasse: 591 g/mol

## Beispiel:

A) Herstellung eines Alkylenoxid / Maleinsäureanhydrid - Copolymerisates über DMC-Katalyse (Präpolymer nach Schritt a))

In einen 2 l Laborautoklaven wurden unter Stickstoffatmosphäre 233,7 g (396 mmol) Polyol A und 0,25 g 85%ige Phosphorsäure gegeben und 30 min. bei Raumtemperatur verrührt (Propellerrührer mit 800 U/min). Nach Zugabe von 0,601 g DMC-Katalysator wurde der Inhalt des Autoklaven 30 min. bei 130 °C und Rühren (Propellerrührer) mit 450 U/min bei einem absoluten Druck von 100 bis 120 mbar unter Einleiten von 50 ml Stickstoff pro Minute über einen unter dem Flüssigkeitsspiegel liegenden Verteilerring gestrippt. Bei 130 °C und unter Rühren mit 800 U/min. wurde ein Gemisch aus 156,5 g Propylenoxid und 469,7 g Ethylenoxid über einen Zeitraum von 2,02 h in den Kopfraum des Autoklaven eindosiert, man begann die Dosierung bei einem absoluten Druck von 0,05 bar. Nach einer Nachreaktionszeit von 20 min. wurde auf Raumtemperatur abgekühlt und 0,627 g Vitamin E sowie 77,9 g (794 mmol) Maleinsäureanhydrid in den Autoklaven gegeben. Restsauerstoff wurde nach Schließen des Befüllstutzens bei 25 °C durch viermaliges Aufdrücken von Stickstoff bis zu einem absoluten Druck von 4 bar und anschließendes Ablassen des Überdrucks auf Atmosphärendruck entfernt. Nach Aufheizen auf 80 °C wurde ein Gemisch aus 65,6 g Propylenoxid und 197,0 g Ethylenoxid über einen Zeitraum von 2,65 h in den Kopfraum des Autoklaven eindosiert. Nach einer Nachreaktionszeit von 30 min. wurde das Produkt 30 min. bei 80 °C bei einem absolutem Druck von 10 mbar ausgeheizt; danach wurden 0,214 g Vitamin E zugegeben.

1102,4 g des Zwischenproduktes wurden in einen 2-1 Dreihalskolben gegeben. Nach dreimaligem Evakuieren und Entspannen mit Stickstoff wurden im Stickstoffgegenstrom bei 80 °C 88,2 g Ambosol® zugegeben. Der Kolbeninhalt wurde bei 80°C über einen Zeitraum von 3 h gerührt, danach wurde für weitere 3 h bei 80 °C und einem absoluten Druck von 1 mbar ausgeheizt. Das Ambosol® wurde sodann über Filtration über eine mit Schleichdampf (ca. 100 °C) beheizte Labornutsche abgetrennt.

Das erhaltene Zwischenprodukt wies eine Viskosität von 1854 mPas bei 25 °C, eine OH-Zahl von 38 mg KOH/g und eine Säurezahl von 10 ppm KOH auf. Über Größenausschlusschromatographie wurde eine Polydispersität (Mw/Mn) von 1,38 ermittelt. Eine solch geringe Polydispersität ist mit einem über Polykondensationsreaktion hergestellten ungesättigten Polyester nicht zu erreichen, denn die Molmassen solchermaßen hergestellter Produkte unterliegen der Schultz-Flory-Verteilung wohingegen die nach dem erfindungsgemäßen Verfahren über DMC-Katalyse hergestellten Zwischenprodukte deutlich engere Poisson-Verteilungen aufweisen.

B) Umsetzung des Zwischenproduktes aus A) mit N-Butylamin (Schritt b))

100 g (33,8 mmol) des Zwischenproduktes aus Schritt A) wurden in einen 500 ml 4-Halskolben mit aufgesetztem Rückflusskühler, Innenthermometer und Magetrührer gegeben. Nach dreimaligem Evakuieren und Entspannen mit Stickstoff wurden im Stickstoffgegenstrom 5,0644 g (69,2 mmol) N-Butylamin zugegeben. Innerhalb weniger Minuten stieg die Kolbeninnentemperatur von 26 °C auf 34 °C an. Nach 30 min. wurde die Kolbeninnentemperatur mittels Heizpilz auf 60 °C angehoben und unter Rühren bei dieser Temperatur 4 h gehalten.

Die Viskosität des Produkts lag bei 1635 mPas bei 25 °C. Die gemessene "OH-Zahl" betrug 73,4 mg KOH / g, wobei die gemessene "OH-Zahl" in diesem speziellen Fall die Summe aus Aminzahl und der eigentlichen OH-Zahl ist. Die Aminzahl lag bei 35 mg KOH / g, was in etwa der Hälfte der gemessenen "OH-Zahl" entspricht. Die Stöchiometrie des MSA-Einbaus in der Vorstufe war so gewählt worden, dass 1 MSA pro OH-Gruppe eingesetzt wurde. Das Ergebnis zeigt, dass nahezu alle Doppelbindungen umgesetzt wurden.

## Patentansprüche

**1.** Verfahren zur Herstellung eines Hydroxy-Aminopolymers umfassend die Schritte:

a) Umsetzen einer wenigstens ein Zerewitinoff-aktives H-Atom tragenden H-funktionellen Starterverbindung mit einem ungesättigten zyklischen Carbonsäureanhydrid und wenigstens einer Alkylenoxidverbindung zum Erhalt eines Hydroxylgruppen tragenden Präpolymers,
b) Addition eines primären Amins und/oder von Ammoniak an die Doppelbindungen des nach Schritt a) erhaltenen Hydroxylgruppen tragenden Präpolymers zum Erhalt des Hydroxy-Aminopolymers,

wobei
die Umsetzung der H-funktionellen Starterverbindung mit dem ungesättigten zyklischen Carbonsäureanhydrid und/oder die Addition der Alkylenoxidverbindung unter Einsatz eines Doppelmetallcyanid-Katalysators (DMC-Katalysator) durchgeführt wird,
die H-funktionelle Starterverbindung 1 bis 35 Zerewitinoff-aktive H-Atome aufweist,
die Alkylenoxidverbindung ausgewählt ist aus solchen mit 2 bis 24 Kohlenstoffatomen,
das Stoffmengenverhältnis zwischen der Alkylenoxidverbindung und dem Carbonsäureanhydrid wenigstens 1,1 : 1 beträgt
und das primäre Amin ausgewählt ist aus aliphatischen, cycloaliphatischen und / oder araliphatischen Mono- und Diaminen mit wenigstens einer primären Aminogruppe und ggf. Hydroxygruppen.

**2.** Verfahren nach Anspruch 1, **dadurch gekennzeichnet, dass** die H-funktionelle Starterverbindung eine zahlenmittlere Molmasse von 17 bis 1200 g/mol aufweist.

**3.** Verfahren nach einem der vorstehenden Ansprüche, **dadurch gekennzeichnet, dass** das ungesättigte cyclische Carbonsäureanhydrid ausgewählt ist aus ungesättigten cyclischen Dicarbonsäureanhydriden.

**4.** Verfahren nach einem der vorstehenden Ansprüche, **dadurch gekennzeichnet, dass** wenigstens ein weiteres Co-Monomer im Schritt a) umgesetzt wird, welches ausgewählt ist aus Lactonen, Lactiden, gesättigten bzw. aromatischen cyclischen Carbonsäureanhydriden, cyclische Carbonaten und / oder Kohlendioxid.

**5.** Verfahren nach einem der vorstehenden Ansprüche, **dadurch gekennzeichnet, dass** das Stoffmengenverhältnis zwischen dem Carbonsäureanhydrid und der Zahl der Zerewitinoff-aktiven H-Atome der H-funktionellen Starterverbindung 1 : 1 bis 1,5 : 1 beträgt.

**6.** Verfahren nach einem der vorstehenden Ansprüche, **dadurch gekennzeichnet, dass** zunächst die H-funktionelle Starterverbindung und der DMC-Katalysator vorgelegt und anschließend die Alkylenoxidverbindung, das cyclische

ungesättigte Carbonsäureanhydrid sowie gegebenenfalls das weitere Co-Monomer hinzugefügt werden.

**7.** Verfahren nach einem der Ansprüche 1 bis 5, **dadurch gekennzeichnet, dass** zunächst die H-funktionelle Starterverbindung und der DMC-Katalysator vorgelegt, Alkylenoxidverbindung sowie gegebenenfalls weiteres Co-Monomer zudosiert werden und anschließend das ungesättigte cyclische Carbonsäureanhydrid zugegeben wird, wobei nach erfolgter Zugabe des ungesättigten cyclischen Carbonsäureanhydrids nochmals Alkylenoxidverbindung sowie gegebenenfalls weiteres Co-Monomer zudosiert wird.

**8.** Verfahren nach einem der Ansprüche 1 bis 5, **dadurch gekennzeichnet, dass** in Schritt a) ein H-funktionelles Starterpolyol und der DMC-Katalysator vorgelegt und die H-funktionelle Starterverbindung kontinuierlich gemeinsam mit dem Alkylenoxid und dem ungesättigten cyclischen Carbonsäureanhydrid zugeführt werden, wobei das H-funktionelle Starterpolyol eine OH-Zahl im Bereich von 3 bis 1000 mg KOH/g aufweist.

**9.** Verfahren nach Anspruch 8, **dadurch gekennzeichnet, dass** als H-funktionelles Starterpolyol ein gemäß Schritt a) hergestelltes Zwischenprodukt eingesetzt wird.

**10.** Verfahren nach einem der Ansprüche 8 und 9, **dadurch gekennzeichnet, dass** die Dosierung des cyclischen Carbonsäureanhydrids vor der Dosierung der Alkylenoxidverbindung beendet und in einer abschließenden, ausschließlich Alkylenoxid sowie gewünschtenfalls ein Co-Monomer umfassenden Dosierung mehr als 1 mol Alkylenoxidverbindung pro mol Zerewitinoff-aktivem Wasserstoff zudosiert wird.

**11.** Verfahren nach einem der Ansprüche 8 und 9, **dadurch gekennzeichnet, dass** in Schritt a) ein Starterpolyol, eine Teilmenge des DMC-Katalysators im Reaktorsystem vorgelegt, die H-funktionelle Verbindung, weiterer DMC-Katalysator gemeinsam mit dem Alkylenoxid und dem ungesättigten cyclischen Carbonsäureanhydrid kontinuierlich zugeführt werden, wobei das hierbei entstandene Reaktionsprodukt des Schritts a) kontinuierlich dem Reaktorsystem nach einer vorwählbaren mittleren Verweilzeit entnommen wird.

**12.** Verfahren nach einem der vorstehenden Ansprüche, bei dem das primäre Amin ausgewählt ist aus Methylamin, Ethylamin, 1-Aminopropan, 1-Aminobutan, Isobutylamin, 1-Aminohexan, 2-Ethyl-1-Aminohexan, Dodecylamin, Octadecyl-amin, Cyclohexylamin, Benzylamin, N-Methylethylendiamin, N-Methylpropylendiamin, N-(2-Aminoethyl)-piperazin und 3-Amino-1,2,4-triazol, N,N-dimethylethylendiamin, N,N-dimethyl-1,3-diaminopropan, N,N-dimethyl-1,8-diaminooctan, N,N-dimethyl-1,4-diaminocyclohexan, Isopropanolamin, und Ethanolamin.

**13.** Verfahren nach Anspruch 12, bei dem das primäre Amin ausgewählt ist aus Ethylamin, 1-Aminobutan, Dodecylamin, Cyclohexylamin, Benzylamin, N,N-dimethyl-1,3-diaminopropan, Ethanolamin und Isopropanolamin.

**14.** Hydroxy-Aminopolymer, erhältlich nach einem Verfahren gemäß einem der Ansprüche 1 bis 13, wobei das Stoffmengenverhältnis zwischen der Alkylenoxidverbindung und dem Carbonsäureanhydrid wenigstens 1,1 : 1 beträgt;**dadurch gekennzeichnet, dass** das Hydroxy-Aminopolymer die allgemeine Formel (VII) besitzt

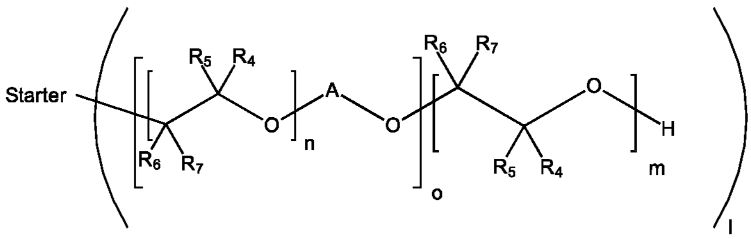

(VII)

,

wobei

"Starter" für das Radikal der H-funktionellen Starterverbindung steht,
A für eine Aspartatgruppe folgender Struktur der Formel (VIIIa) oder (VIIIb) steht

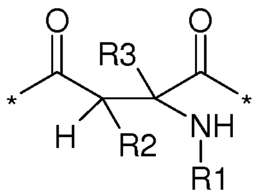

(VIIIa)

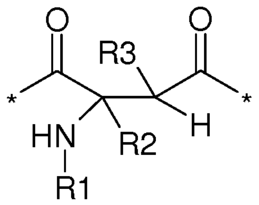

(VIIIb),

in der

R2 und, R3 unabhängig voneinander für Wasserstoff oder einen aliphatischen oder aromatischen Rest stehen und R2 und R3 auch Bestandteil eines cycloaliphatischen Ringsystems sein können,
R1 für Wasserstoff oder einen aliphatischen, cycloaliphatischen oder aromatischen Rest steht, der auch Heteroatome, insbesondere Stickstoffatome oder Sauerstoffatome sowie Hydroxygruppen enthalten kann,
R4, R5, R6 und R7 unabhängig voneinander für Wasserstoff oder einen aliphatischen oder aromatischen Rest stehen und R5 und R6 auch Bestandteil eines cycloaliphatischen Ringsystems sein können,
l für die Zahl der Zerewitinoff-aktiven Wasserstoffatome der H-funktionellen Starterverbindung steht,
m, n und o unabhängig voneinander und ganzzahlig sind, wobei n, o = 0 oder $\geq$ 1 und m $\geq$ 2 sind,

wobei die Äquivalentmolmasse der in der Formel VII gezeigten Struktur den Wert 18900 g/mol nicht übersteigt.

**15.** Polyurethanharnstoffpolymer, erhältlich durch Umsetzung eines Polyisocyanats mit einem Hydroxy-Aminopolymer nach Anspruch 14.

**16.** Verwendung eines Hydroxy-Aminopolymers nach Anspruch 14zur Herstellung eines Polyurethanharnstoffpolymers.

## Claims

**1.** Process for preparing a hydroxy-amino polymer, comprising the steps of:

a) reacting an H-functional starter compound carrying at least one Zerewitinoff-active H atom with an unsaturated cyclic carboxylic anhydride and with at least one alkylene oxide compound, to give a prepolymer carrying hydroxyl groups,
b) addition-reacting a primary amine and/or ammonia onto the double bonds of the hydroxyl-carrying prepolymer obtained by step a), to give the hydroxy-amino polymer,

where
the reaction of the H-functional starter compound with the unsaturated cyclic carboxylic anhydride and/or the addition reaction of the alkylene oxide compound are/is carried out using a double metal cyanide catalyst (DMC catalyst),
the H-functional starter compound has 1 to 35 Zerewitinoff-active H atoms,
the alkylene oxide compound is selected from those having 2 to 24 carbon atoms,
the amount-of-substance ratio between the alkylene oxide compound and the carboxylic anhydride is at least 1.1:1,
and the primary amine is selected from aliphatic, cycloaliphatic and/or araliphatic monoamines and diamines having at least one primary amino group and optionally hydroxyl groups.

**2.** Process according to Claim 1, **characterized in that** the H-functional starter compound has a number-average molar mass of 17 to 1200 g/mol.

3. Process according to either of the preceding claims, **characterized in that** the unsaturated cyclic carboxylic anhydride is selected from unsaturated cyclic dicarboxylic anhydrides.

4. Process according to any of the preceding claims, **characterized in that** at least one further comonomer is reacted in step a), and is selected from lactones, lactides, saturated and/or aromatic cyclic carboxylic anhydrides, cyclic carbonates and/or carbon dioxide.

5. Process according to any of the preceding claims, **characterized in that** the amount-of-substance ratio between the carboxylic anhydride and the number of Zerewitinoff-active H atoms in the H-functional starter compound is 1:1 to 1.5:1.

6. Process according to any of the preceding claims, **characterized in that** initially the H-functional starter compound and the DMC catalyst are introduced and subsequently the alkylene oxide compound, the cyclic unsaturated carboxylic anhydride, and optionally the further comonomer are added.

7. Process according to any of Claims 1 to 5, **characterized in that** initially the H-functional starter compound and the DMC catalyst are introduced, alkylene oxide compound and also optionally further comonomer are metered in, and subsequently the unsaturated cyclic carboxylic anhydride is added, the addition of the unsaturated cyclic carboxylic anhydride being followed by further metered addition of alkylene oxide compound and also, optionally, of further comonomer.

8. Process according to any of Claims 1 to 5, **characterized in that** in step a) an H-functional starter polyol and the DMC catalyst are introduced initially and the H-functional starter compound is supplied continuously together with the alkylene oxide and the unsaturated cyclic carboxylic anhydride, with the H-functional starter polyol having an OH number in the range from 3 to 1000 mg KOH/g.

9. Process according to Claim 8, **characterized in that** an intermediate prepared according to step a) is used as H-functional starter polyol.

10. Process according to either of Claims 8 and 9, **characterized in that** the metering of the cyclic carboxylic anhydride is ended before the metering of the alkylene oxide compound and in a concluding metering, comprising exclusively alkylene oxide and also, if desired, a comonomer, more than 1 mol of alkylene oxide compound per mole of Zerewitinoff-active hydrogen is metered in.

11. Process according to either of Claims 8 and 9, **characterized in that** in step a) a starter polyol and a portion of the DMC catalyst are introduced initially in the reactor system, the H-functional compound and further DMC catalyst are supplied continuously together with the alkylene oxide and the unsaturated cyclic carboxylic anhydride, and the step a) reaction product formed in this procedure is withdrawn continuously from the reactor system after a preselectable average residence time.

12. Process according to any of the preceding claims, wherein the primary amine is selected from methylamine, ethylamine, 1-aminopropane, 1-aminobutane, isobutylamine, 1-aminohexane, 2-ethyl-1-aminohexane, dodecylamine, octadecylamine, cyclohexylamine, benzylamine, N-methylethylenediamine, N-methylpropylenediamine, N-(2-aminoethyl)piperazine and 3-amino-1,2,4-triazole, N,N-dimethylethylenediamine, N,N-dimethyl-1,3-diaminopropane, N,N-dimethyl-1,8-diaminooctane, N,N-dimethyl-1,4-diaminocyclohexane, isopropanolamine, and ethanolamine.

13. Process according to Claim 12, wherein the primary amine is selected from ethylamine, 1-aminobutane, dodecylamine, cyclohexylamine, benzylamine, N,N-dimethyl-1,3-diaminopropane, ethanolamine, and isopropanolamine.

14. Hydroxy-amino polymer obtainable by a process according to any of Claims 1 to 13, where the amount-of-substance ratio between the alkylene oxide compound and the carboxylic anhydride is at least 1.1:1, **characterized in that** the hydroxy-amino polymer possesses the general formula (VII)

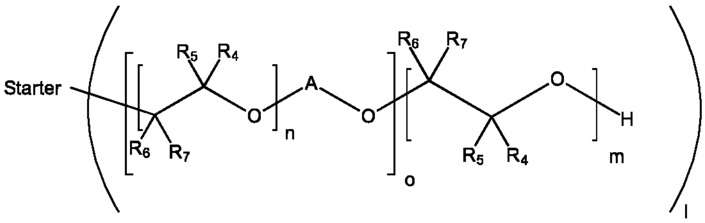

(VII)

where

"Starter" stands for the radical of the H-functional starter compound,
A is an aspartate group of the following structure, of the formula (VIIIa) or (VIIIb)

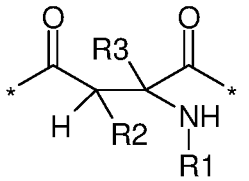

(VIIIa)

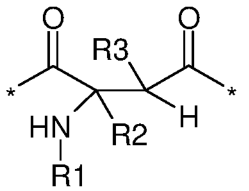

(VIIIb),

in which

R2 and, R3 independently of one another are hydrogen or an aliphatic or aromatic radical, and R2 and R3 may also be part of a cycloaliphatic ring system,
R1 is hydrogen or an aliphatic, cycloaliphatic, or aromatic radical, which may also contain heteroatoms, more particularly nitrogen atoms or oxygen atoms, and also hydroxyl groups, R4, R5, R6, and R7 independently of one another are hydrogen or an aliphatic or aromatic radical, and R5 and R6 may also be part of a cycloaliphatic ring system,
1 is the number of Zerewitinoff-active hydrogen atoms in the H-functional starter compound,
m, n, and o are independent of one another and are integral, with n, o = 0 or ≥ 1 and m ≥ 2,

where the equivalent molar mass of the structure shown in the formula VII does not exceed the value of 18 900 g/mol.

**15.** Polyurethaneurea polymer obtainable by reacting a polyisocyanate with a hydroxy-amino polymer according to Claim 14.

**16.** Use of a hydroxy-amino polymer according to Claim 14 for preparing a polyurethaneurea polymer.

**Revendications**

**1.** Procédé de préparation d'un hydroxyaminopolymère, comprenant les étapes de :

a) transformation d'un composé de départ à fonctionnalité H portant au moins un atome d'H actif selon Zerewitinoff avec un anhydride d'acide carboxylique cyclique insaturé et au moins un composé de type oxyde d'alkylène pour obtenir un prépolymère portant des groupes hydroxyle,
b) addition d'une amine primaire et/ou d'ammoniac sur les doubles liaisons du prépolymère portant des groupes

hydroxyle obtenu selon l'étape a) pour obtenir l'hydroxyaminopolymère

la transformation du composé de départ à fonctionnalité H avec l'anhydride d'acide carboxylique cyclique insaturé et/ou l'addition du composé de type oxyde d'alkylène étant effectuée(s) tout en utilisant un catalyseur de type cyanure métallique double (catalyseur DMC),
le composé de départ à fonctionnalité H présentant 1 à 35 atomes d'H actif selon Zerewitinoff,
le composé de type oxyde d'alkylène étant choisi parmi ceux comprenant 2 à 24 atomes de carbone,
le rapport des quantités entre le composé de type oxyde d'alkylène et l'anhydride d'acide carboxylique étant d'au moins 1,1:1
et l'amine primaire étant choisie parmi les monoamines et les diamines aliphatiques, cycloaliphatiques et/ou aliphatiques présentant au moins un groupe amino primaire et le cas échéant des groupes hydroxy.

2. Procédé selon la revendication 1, **caractérisé en ce que** le composé de départ à fonctionnalité H présente une masse molaire moyenne en nombre de 17 à 1200 g/mole.

3. Procédé selon l'une quelconque des revendications précédentes, **caractérisé en ce que** l'anhydride d'acide carboxylique cyclique insaturé est choisi parmi les anhydrides d'acides dicarboxyliques cycliques insaturés.

4. Procédé selon l'une quelconque des revendications précédentes, **caractérisé en ce qu'**au moins un comonomère supplémentaire est transformé dans l'étape a), qui est choisi parmi les lactones, les lactides, les anhydrides d'acides carboxyliques cycliques saturés ou aromatiques, les carbonates cycliques et/ou le dioxyde de carbone.

5. Procédé selon l'une quelconque des revendications précédentes, **caractérisé en ce que** le rapport des quantités entre l'anhydride d'acide carboxylique et le nombre d'atomes d'H actif selon Zerewitinoff du composé de départ à fonctionnalité H est de 1:1 à 1,5:1.

6. Procédé selon l'une quelconque des revendications précédentes, **caractérisé en ce que** le composé de départ à fonctionnalité H et le catalyseur DMC sont d'abord disposés au préalable et le composé de type oxyde d'alkylène, l'anhydride d'acide carboxylique insaturé cyclique ainsi que le cas échéant le comonomère supplémentaire sont ajoutés ensuite.

7. Procédé selon l'une quelconque des revendications 1 à 5, **caractérisé en ce que** le composé de départ à fonctionnalité H et le catalyseur DMC sont d'abord disposés au préalable, le composé de type oxyde d'alkylène ainsi que le cas échéant le comonomère supplémentaire sont ajoutés en dosant et, ensuite, l'anhydride d'acide carboxylique cyclique insaturé est ajouté, du composé de type oxyde d'alkylène ainsi que le cas échéant du comonomère supplémentaire étant de nouveau ajoutés en dosant après la fin de l'addition de l'anhydride d'acide carboxylique cyclique insaturé.

8. Procédé selon l'une quelconque des revendications 1 à 5, **caractérisé en ce que**, dans l'étape a), un polyol de départ à fonctionnalité H et le catalyseur DMC sont disposés au préalable et le composé de départ à fonctionnalité H est ajouté en continu ensemble avec l'oxyde d'alkylène et l'anhydride d'acide carboxylique cyclique insaturé, le polyol de départ à fonctionnalité H présentant un indice d'OH dans la plage de 3 à 1000 mg de KOH/g.

9. Procédé selon la revendication 8, **caractérisé en ce qu'**on utilise, comme polyol de départ à fonctionnalité H, un produit intermédiaire préparé selon l'étape a).

10. Procédé selon l'une quelconque des revendications 8 et 9, **caractérisé en ce que** le dosage de l'anhydride d'acide carboxylique cyclique se termine avant le dosage du composé de type oxyde d'alkylène et, lors d'un dosage final comprenant uniquement de l'oxyde d'alkylène ainsi que le cas échéant un comonomère, plus de 1 mole de composé d'oxyde d'alkylène par mole d'hydrogène actif selon Zerewitinoff est ajoutée en dosant.

11. Procédé selon l'une quelconque des revendications 8 et 9, **caractérisé en ce que**, dans l'étape a), un polyol de départ et une quantité partielle du catalyseur DMC sont disposés au préalable dans le système de réacteur, le composé à fonctionnalité H et un supplément de catalyseur DMC sont ajoutés en continu ensemble avec l'oxyde d'alkylène et l'anhydride d'acide carboxylique cyclique insaturé, le produit de réaction ainsi formé de l'étape a) étant prélevé en continu du système de réacteur après une durée de séjour moyenne pouvant être présélectionnée.

12. Procédé selon l'une quelconque des revendications précédentes, dans lequel l'amine primaire est choisie parmi la

méthylamine, l'éthylamine, le 1-aminopropane, le 1-aminobutane, l'isobutylamine, le 1-aminohexane, le 2-éthyl-1-aminohexane, la dodécylamine, l'octadécylamine, la cyclohexylamine, la benzylamine, la N-méthyléthylènediamine, la N-méthylpropylènediamine, la N-(2-aminoéthyl)-pipérazine et le 3-amino-1,2,4-triazol, la N,N-diméthyléthylènediamine, le N,N-diméthyl-1,3-diaminopropane, le N,N-diméthyl-1,8-diaminooctane, le N,N-diméthyl-1,4-diaminocyclohexane, l'isopropanolamine et l'éthanolamine.

**13.** Procédé selon la revendication 12, dans lequel l'amine primaire est choisie parmi l'éthylamine, le 1-aminobutane, la dodécylamine, la cyclohexylamine, la benzylamine, le N,N-diméthyl-1,3-diaminopropane, l'éthanolamine et l'isopropanolamine.

**14.** Hydroxyaminopolymère, pouvant être obtenu selon un procédé selon l'une quelconque des revendications 1 à 13, le rapport de quantités entre le composé de type oxyde d'alkylène et l'anhydride d'acide carboxylique étant d'au moins 1,1:1, **caractérisé en ce que** l'hydroxyaminopolymère présente la formule générale (VII)

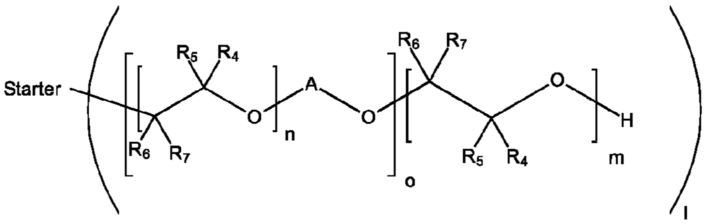

(VII)

dans laquelle

"Starter" représente le radical du composé de départ à fonctionnalité H,
A représente un groupe aspartate de la structure suivante de formule (VIIIa) ou (VIIIb)

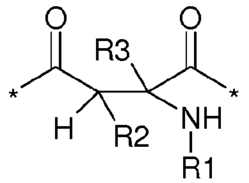

(VIIIa)

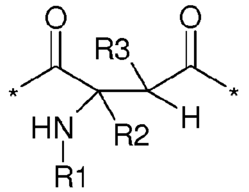

(VIIIb),

dans lesquelles

R2 et R3 représentent, indépendamment l'un de l'autre, hydrogène ou un radical aliphatique ou aromatique et R2 et R3 peuvent également faire partie d'un système cyclique cycloaliphatique
R1 représente hydrogène ou un radical aliphatique, cycloaliphatique ou aromatique, qui peut également contenir des hétéroatomes, en particulier des atomes d'azote ou des atomes d'oxygène ainsi que des groupes hydroxy,
R4, R5, R6 et R7 représentent, indépendamment les uns des autres, hydrogène ou un radical aliphatique ou aromatique et R5 et R6 peuvent également faire partie d'un système cyclique cycloaliphatique
l représente le nombre d'atomes d'hydrogène actif selon Zerewitinoff du composé de départ à fonctionnalité H,
m, n et o sont indépendants les uns des autres et des nombres entiers, n, o = 0 ou ≥ 1 et m ≥ 2,

la masse molaire équivalente de la structure montrée dans la formule VII n'étant pas supérieure à 18.900 g/mole.

**15.** Polymère de polyuréthane-urée pouvant être obtenu par transformation d'un polyisocyanate avec un hydroxyaminopolymère selon la revendication 14.

**16.** Utilisation d'un hydroxyaminopolymère selon la revendication 14 pour la préparation d'un polymère de polyuréthane-urée.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5739192 A1
- US 5597390 A1
- US 20050171002 A1
- DE 19616984 A1
- DE 19508308 A1
- WO 2010090345 A1
- JP 2009022753 A
- JP 04089860 A
- US 4874837 A1
- US 5554687 A
- EP 1525244 A1
- US 3404109 A1
- US 3829505 A1
- US 3941849 A1
- US 5158922 A1
- US 5470813 A1
- EP 700949 A1
- EP 743093 A1
- EP 761708 A1
- WO 9740086 A1
- WO 9816310 A1
- WO 0047649 A1
- EP 10163170
- US A5158922 A1
- JP 4145123 A
- WO 0139883 A1
- WO 0180994 A1
- US 4987271 A1
- DE 3132258 A1
- EP 406440 A1
- US 5391722 A1
- US 5099075 A1
- US 4721818 A1
- US 4877906 A1
- EP 385619 A1
- EP 0007502 A1


### In der Beschreibung aufgeführte Nicht-Patentliteratur


- Ullmann's Encyclopedia of Industrial Chemistry. 1992, vol. B4, 167
- Handbuch Apparate; Vulkan-Verlag Essen. 1990, 188-208
- **VON W. SIEFKEN.** *Justus Liebigs Annalen der Chemie,* vol. 562, 75-136